(19) **Europäisches Patentamt / European Patent Office / Office européen des brevets**

(11) **EP 2 170 432 B1**

(12) **EUROPEAN PATENT SPECIFICATION**

(45) Date of publication and mention of the grant of the patent:
**06.11.2013 Bulletin 2013/45**

(21) Application number: **08770799.8**

(22) Date of filing: **12.06.2008**

(51) Int Cl.:
*A61M 5/158* (2006.01)     *A61M 5/32* (2006.01)
*A61M 25/00* (2006.01)

(86) International application number:
**PCT/US2008/066667**

(87) International publication number:
**WO 2008/157212 (24.12.2008 Gazette 2008/52)**

(54) **FLEXIBLE MEDICAL CANNULA**

FLEXIBLE MEDIZINISCHE KANÜLE

CANULE MEDICALE FLEXIBLE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **15.06.2007   US 944329 P**
**29.10.2007   US 983530 P**
**30.10.2007   US 983651 P**
**31.10.2007   US 984066 P**

(43) Date of publication of application:
**07.04.2010   Bulletin 2010/14**

(73) Proprietors:
• **Lifescan, Inc.**
**Milpitas, CA 94066 (US)**
• **Cordis Corporation**
**Miami Lakes, FL (US)**

(72) Inventors:
• **KRULEVITCH, Peter**
**Pleasanton, CA 94566 (US)**

• **OLSON, Lorin, P.**
**Scotts Valley, CA 95066 (US)**
• **SIEH, Zara**
**Pleasanton, CA 94566 (US)**
• **SAVAGE, Donna**
**Rolling Hills Estates, CA 90274 (US)**
• **CICHOCKI, Frank, R.**
**Easton, PA 18045 (US)**

(74) Representative: **Tunstall, Christopher Stephen et al**
**Carpmaels & Ransford LLP**
**One Southampton Row**
**London WC1B 5HA (GB)**

(56) References cited:
**EP-A- 0 615 768        WO-A-02/05866**
**WO-A-98/17337        WO-A-02/081013**
**WO-A-2005/068000    US-A1- 2002 072 720**

**Description**

**BACKGROUND OF THE INVENTION**

Field of the Invention
The present invention relates, in general, to medical devices and, in particular, to flexible medical device conduits, i.e. flexible medical cannulas, and associated insertion devices and methods.
Description of Related Art
A variety of medical devices employ conduits for accessing body target sites in order to perform diagnostic, therapeutic, and surgical procedures. For example, flexible cannulas inserted into a skin target site by rigid needles are conventionally employed for the infusion of therapeutic agents (e.g., insulin). Such cannulas are known from e.g. WO 02/081013 or WO 98/17337. WO 02/05866 teaches a catheter.

**SUMMARY OF INVENTION**

It is an objet of the present invention defined in claim 1 to provide a flexible medical cannula comprising: an elongated framework formed from a flexible material, the elongated framework having: a body portion; a sharp head; a distal end; and a proximal end; and a flexible tube at least partially jacketing the elongated framework between the distal end and the proximal end, wherein the sharp head is disposed at the distal end; wherein the elongated framework and flexible tube define at least one conduit therebetween with at least one opening therealong for permitting fluid to exit through said conduit; and wherein the sharp head is configured for insertion into a target site.
A method for manufacturing a flexible medical device conduit is presented herein, the method comprising: forming an elongated framework of flexible material; creating a sharp head s f3 it distal end of the elongated strip; and jacketing the elongated framework with a flexible tube such that the flexible tube and the elongated framework define at least one conduit, therebetween.
Further, a flexible conduit insertion medical device may comprise: an elongated framework formed from a flexible material, the elongated framework having: a body portion; a sharp head; a distal end; and a proximal end; and a flexible tube at least partially jacketing the elongated framework between the distal end and the proximal end, wherein the sharp head is disposed at the distal end; wherein the elongated framework and flexible tube define at least one conduit therebetween with at least one opening therealong; and wherein the sharp head is configured for target site insertion; and an insertion mechanism operatively connected to, and integrated with, the flexible medical device conduit, the insertion mechanism configured to insert a portion of the flexible medical device conduit, including at least the sharp head and the opening, into a target site.
Finally, a method for inserting a flexible medical conduit into a user's target site is presented herein, the method comprising: adhering a flexible conduit insertion medical device to a target site, the flexible conduit insertion medical device including: an elongated framework formed from a flexible material, the elongated framework having: a body portion; a sharp head; a distal end; and a proximal end; and a flexible tube at least partially jacketing the elongated framework between the distal end and the proximal end, wherein the sharp head is disposed at the distal end; wherein the elongated framework and flexible tube define at least one conduit therebetween with at least one opening therealong; and wherein the sharp head is configured for insertion into a target site; and an insertion mechanism operatively connected to, and integrated with, the flexible medical device conduit, the insertion mechanism configured to insert a portion of the flexible medical device conduit, including at least the sharp head and the opening, into the skin target site; and inserting the flexible medical device conduit into the targer site by action of the insertion mechanism.

**BRIEF DESCRIPTION OF THE DRAWINGS**

The novel features of the invention are set forth with particularity in the appended claims. A better understanding of the features and advantages of the present invention will be obtained by reference to the following detailed description that sets forth illustrative embodiment, in which the principles of the invention are utilized, and the accompanying drawings, in which like numerals indicate like elements, of which:

FIG. 1A is a simplified cross-sectional depiction of a portion of a flexible medical device conduit according to an embodiment of the present invention;
FIG. 1B is a simplified cross-sectional depiction of the portion of a flexible medical device conduit of FIG. 1A taken along line B-B of FIG. 1A;
FIG. 1C is a simplified cross-sectional depiction of the portion of a flexible conduit of FIG. 1A taken along line C-C of FIG. 1A;
FIG. 2 is a simplified cross-sectional depiction of a flexible medical device conduit according to another embodiment of the present invention;

FIG. 3 is a simplified cross-sectional depiction of a flexible medical device conduit according to yet another embodiment of the present invention;

FIG. 4 is a simplified cross-sectional depiction of a flexible medical device conduit according to still another embodiment of the present invention:

FIG. 5 is a simplified perspective view of a flexible medical device conduit according to an embodiment of the present invention with equivalent flexibility in two directions;

FIGs. 6A and 6B are simplified depictions, side and cross-sectional views along line B-B of FIG. 6A respectively, of an elongated strip with a channel therein as can be employed in embodiments of present invention;

FIG. 7 is a simplified cross-sectional depiction of an elongated strip as can be employed in embodiments of the present invention;

FIG. 8 is a simplified cross-sectional depiction of another elongated strip as can be employed in embodiments of the present invention;

FIG. 9 is a simplified depiction of yet another elongated strip as can be employed in embodiments of the present invention;

FIG. 10 is a simplified depiction of a portion of still another elongated strip as can be employed in embodiments of the present invention;

FIGs. 11A-11D are simplified depictions of various elongated strip configurations as can be employed in embodiments of the present invention:

FIGs. 12A and 12B are simplified depictions of steps in an isotropic etching process as can be employed in methods to manufacture flexible medical device conduits;

FIG. 13 is a simplified enlarged view of the distal end of a flexible medical device conduit having holes along its lenght according to an embodiment of the present invention;

FIGs. 14A, 14B and 14C are simplified views of a flexible conduit insertion medical device usable with an embodiment of the present invention;

FIG. 15A - 15C are simplified illustrations of various states of a flexible conduit insertion medical device according to an embodiment of the present invention;

FIGs. 16A - 16H are various simplified views of a flexible conduit insertion medical device;

FIG. 17 is a simplified cross-sectional view of a connector that may be used in medical device embodiments of the present invention;

FIGs. 18A and 18B are simplified cross-sectional views of another connector that may be used in medical device embodiments of the present invention;

FIGs. 19A and 19B are simplified cross-sectional views of yet another connector that may be used in medical device embodiments of the present invention;

FIGs. 20A - 20D are simplified views of a flexible conduit insertion medical device;

FIGs. 21A - 21D are simplified views of a medical device according to another embodiment of the present invention;

FIGs. 22A and 22B are simplified views of a flexible conduit insertion medical device before deployment of a flexible conduit;

FIGs. 23A and 23B are simplified views of a flexible conduit insertion medical device before deployment of a flexible conduit;

FIGs. 24A - 24H are various simplified views of a flexible conduit insertion medical device;

FIG. 25 is a simplified depiction of a flexible conduit insertion medical device as can be employed with embodiments of the present invention;

FIG. 26 is a flow diagram depicting stages in a process for manufacturing a flexible medical device conduit according to an embodiment of the present invention; and

FIG. 27 is a flow diagram depicting stages in a process for inserting a flexible medical device conduit into a target site.

## DETAILED DESCRIPTION OF ILLUSTRATIVE EMBODIMENTS

The following detailed description should be read with reference to the drawings, in which like element in different drawings are identically numbered. The drawings, which are not necessarily to scale, depict exemplary embodiments for the purpose of explanation only. The detailed description illustrates by way of example the principles of the invention. This description will clearly enable one skilled in the art to make and use the invention, and describes several embodiments, adaptions, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. In addition, as used herein, the terms "patient," "host," "user" and "subject" refer to any human or animal subject and are not intended to limit the devices or methods to human use, although use of the subject invention in a human patient represents a preferred embodiment.

Flexible medical cannulas according to the presem invention include an elongated framework (such as, for example, an elongated strip with a channel along a longitudinal axis thereof) formed from a flexible material (e.g., Nitinol) with a body portion, a sharp head, a distal end and a proximal end. The flexible medical device conduit also includes a flexible tube at least partially jacketing the elongated framework between the distal end and the proximal end. In addition, the sharp head is disposed at the distal end and is configured for motion into a target site (e.g., subcutaneous insertion into a skin target site). Moreover, the elongated framework and flexible tube define at least one conduit (also referred to herein as a "lumen" or "internal lumen") between the elongated framework and the flexible tube, the conduit having an opening therealong (e.g., an opening at the distal end and/or partially within the sharp head). Further features, characteristics and benefits of such flexible medical device are described below with respect to various drawings.

Flexible medical device conduits according to embodiments of the present invention are beneficial in that, for example, they can be consistantly inserted to a predetermined depth below the skin, are comfortably flexible while being kink-resistant, and have a relatively small cross-sectional area. Flexible medical device conduits according to embodiments of the present invention are an easily and inexpensively manufactured design in comparison to conventional cannulas.

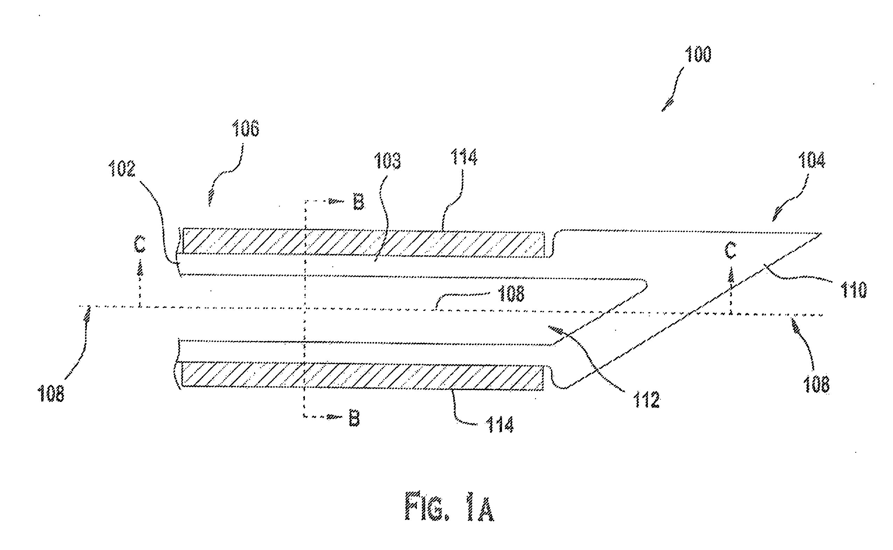

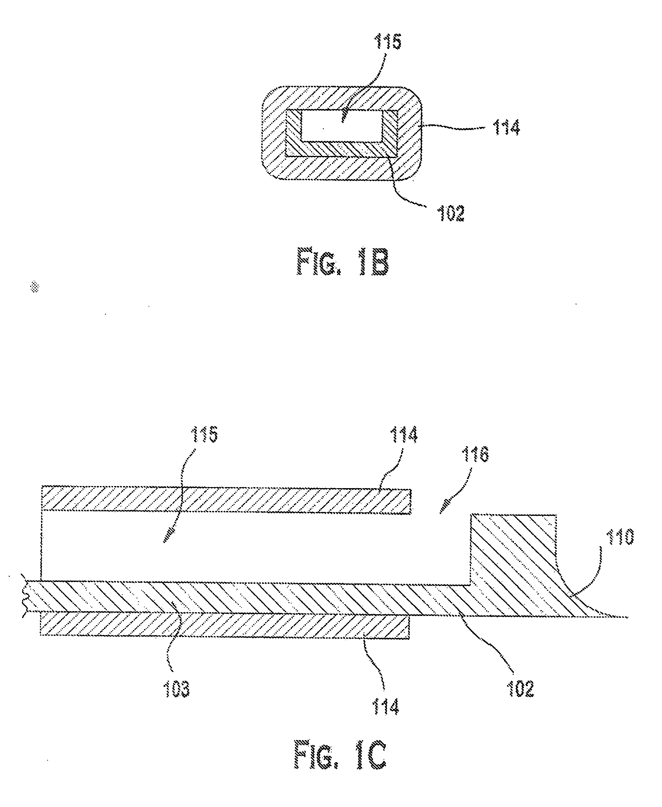

FIGs. 1A, 1B and 1C depict, in a simplified manner, a portion of a flexible medical device conduit 100 according to an embodiment of the present invention. It should be noted that the cross-hatching of FIG. 1A is simplified in comparison to FIGs. 1B and 1C.

Referring to FIGs. 1A through 1C, flexible medical device conduit 100 includes an elongated strip 102 formed from a flexible material (such as a Nitinol, other suitable flexible material, or other suitable superelastic material) with a body portion 103, a distal end 104, a proximal end 106, a longitudinal axis 108 (depicted by a dashed line), a sharp head 110 disposed at distal end 104 and a channel 112. Channel 112 is disposed parallel to (for example, along) the longitudinal axis 108. As described in further detail below, sharp head 110 is configured for subcutaneous skin insertion.

Flexíble medical device conduit 100 also includes a flexible tube 114 at least partially jacketing elongated strip 102 between distal end 104 and proxímal end 106. The elongated strip and flexible tube define a conduit 115 therebetween. Channel 112 extends partially into sharp head 110 such that an opening 116 of conduit 115 is also defined. One skilled in the art will recognize that conduit 115 will, for example, typically have another opening (not shown) at the proximal end thereof.

If desired, flexible tube 114 can extend past proximal end 106 and be configured to provide a fluid-tight connection to associated medical device components (such as infusion device components). Moreover, if desired, flexible medical device conduit 100 can be partially coated with a lubricious material to facilitate insertion into a user's target site (for example, subcutaneous skin insertion).

Since flexible medical device conduits according to embodiments of the present invention can be formed with an elongated framework that is flexible and kink-resistant, they can have a relatively small cross-sectional area as the kink-resistance enables the use of a flexible tube with a relatively a thin wall. It is hypothesized, without being bound, that such small cross-sectional areas result in reduced subcutaneous insertion pain and are more comfortable to wear than conventional polymeric cannulas that are formed with relatively large outside diameters to prevent kinking.

Nitinol employed in embodiments of the present invention can be beneficially pre-processed (also referred to as preprogrammed) using techniques known to one skilled in the are to possess a variety of superelastic charateristics that are also known to those of skill in the art (such as, for example, kink-resistance, the ability to accommodate large loads and the ability to return to an original (preprogrammed) shape following release of mechanically deforming stresses.

Flexible medical device conduit 100 is very flexible, especially when bending such that the open side of channel 112 faces towards (or away from) the center of the radius of curvature, referred to as the flexible bending direction. Moreover, use of superelastic materials provide for flexible medical device conduit 100 to bend considerably without kinking. Flexible medical device conduit 100 is less flexible when bending about an axis that is perpendicular to the flexible bending direction. The amount of flexibility in different directions is governed by the moment of inertia $I$ of the cross section of elongated strip 102, which is given by Equation 1:

$$I = \int y^2 \mathrm{d}A \qquad\qquad (1)$$

In Equation 1, $y$ is the distance perpendicular to the bending axis and $\mathrm{d}A$ is an infinitesimal cross sectional area. Equation 1 dictates that cross sections with large areas far from the bending axis have high moments of inertia and are inflexible, while cross sections in which of the area is close to the bending axis have low moments of inertia and are flexible.

Bending stiffness is proportional to both moment of inertia $I$, which depends on geometry, and Young's modulus E, which is a material property. Polymers tend to have Young's moduli that are much lower than metals. For example, the Young's modulus for Teflon (which can be used to form the flexible tube) is approximately 0.5 GPa, while the Young's modulus for Nitinol (which can be used to form the elongated framework of flexible medical devices according to the present invention) is 35-75 GPa.

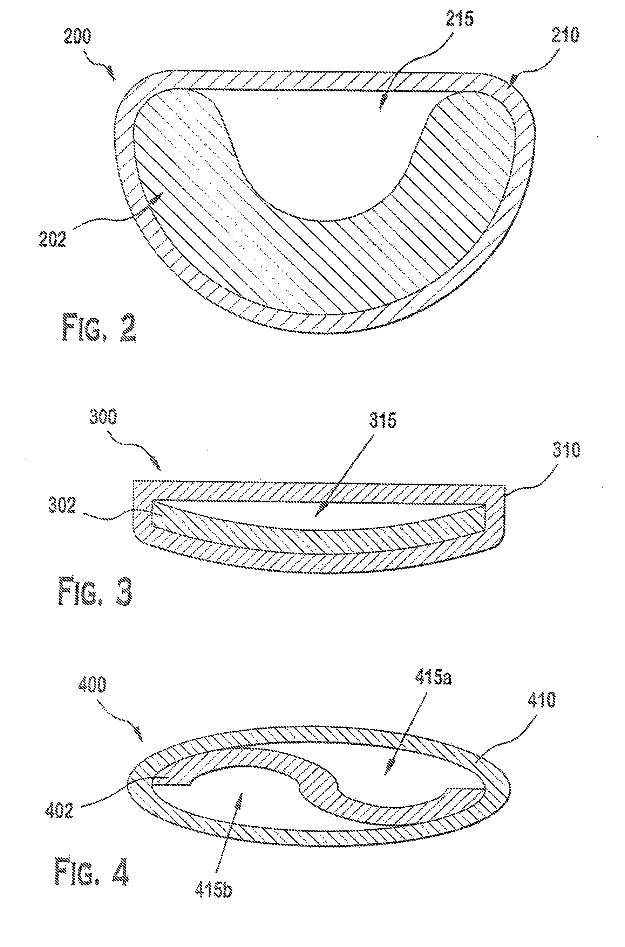

In the embodiment of FIGs. 1A-1C, elongated strip 102 has a C-shaped cross-section (see FIG. 1B in particular). Once apprised of the present disclosure, one skilled in the art will recognize that other suitable elongated strip cross-section shapes can be to control (i.e., predetermine) the amount of flexibility in different directions. In this regard, three examples of elongated strip cross-sections are depicted in FIGs. 2, 3, and 4.

FIG. 2 is a simplified cross-sectional depiction of a flexible medical device conduit 200 (with elongated strip 202, flexible tube 210 and conduit 215) according to another embodiment of the present invention. FIG. 3 is a simplified cross-sectional depiction of a flexible medical device conduit 300 (with elongated strip 302 with a curved cross-section, flexible tube 310 and conduit 315) according to yet another embodiment of the present invention. FIG. 4 is a simplified cross-sectional depíction of a flexible medical device conduit 400 (with elongated strip 402 with an S-shaped cross-section, flexible tube 410 and conduits 415a and 415b) according to still another embodiment of the present invention. Flexible medical device conduit 400 includes two separate internal lumens (e.g., conduits 41 5a and 415b), which may be advantageous for some uses.

Elongated strips 302 and 402, surrounded respectively by flexible tubes 310 and 410, are flatter in cross-section than elongated strips 102 and 202, with most of the area close to the bending axis, which reduces the moment of inertia according to Equation 1. and increases flexibility in the flexible bending direction. Conversely, flexibility perpendicular to the flexible bending direction is decreased for the embodiments of FIGs. 3 and 4 in comparison to the embodiments of FIGs. 1 and 2.

The elongated framework employed in embodiments of the present invention can have a cross section shape that changes along the length of the flexible medical device conduit to provide for varying flexibility along the length.

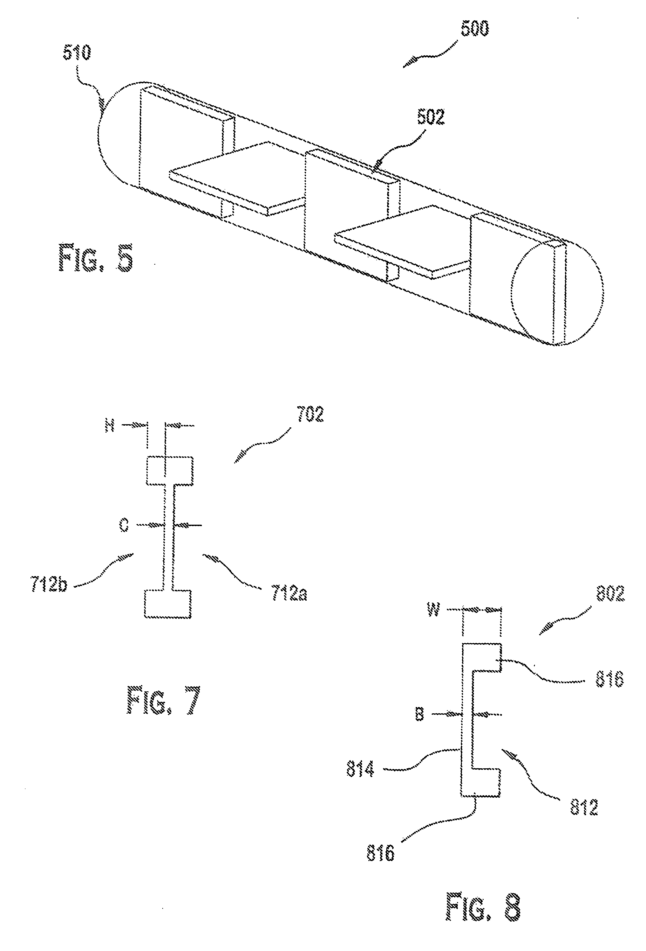

FIG. 5 is a simplified perspective view of a flexible medical device conduit 500 (with elongated framework 502 and flexible tube 510) according to an embodiment of the present invention with equivalent flexibility in two directions. It should be noted that FIG. 5 dspicts elongated framework 502 as it would be seen in the absence of flexible tube 510 and, for simplicity, does not depict the sharp head of flexible medical device conduit 500).

Referring to FIG. 5, elongated framework 502 has a cross-section that varies along the length of flexible medical device conduit 500. Elongated framework 502 can, for example, be made for by crimping a Nitinol strip in two alternating directions. This configuration provides flexibility in two orthogonal directions. The cross-section shape of elongated framework 502 is rectangular, with the orientation of the rectangular cross-section varying along the length of the elongated framework and, thus, along the length of the flexible medical device conduit.

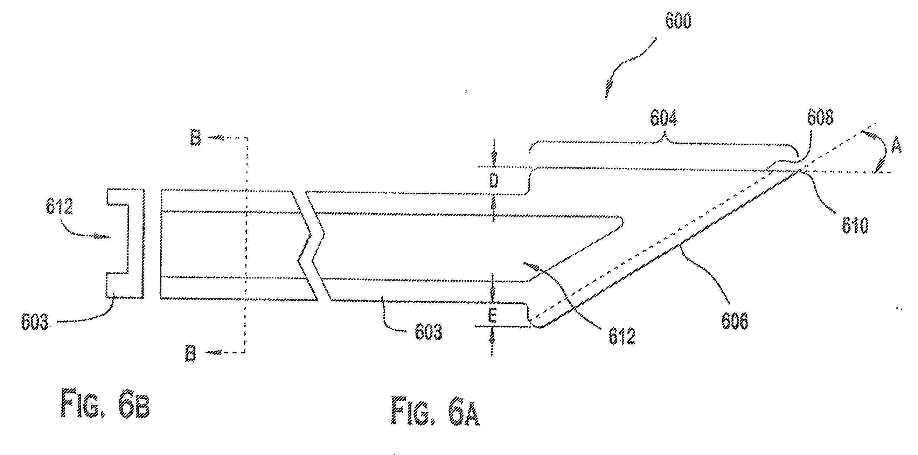

FIGs 6A and 6B are simplified depictions, side and cross-sectional views respectively, of an elongated strip 602 (with body portion 603) formed from Nitinol with a channel 612 therein as can be employed in embodiments of the present invention. Elongated strip 602 further includes a sharp head 604 having a first edge 606 and a second edge 608.

Sharp head 604 is, for example, about 0.03 inches to about 0.05 inches in length. First edge 606 and second edge 608 meet at a tip 610 to form a tip angle A. One or both of the first edge 606 and the second edge 608 can be sharp to provide for subcutaneous insertion of sharp head 605. FIG. 6A illustrates an embodiment in which only first edge 606 is sharp (as indicated by the dashed line running parallel to first edge 606).

**Example 1:** Insertion Force Comparative Study

A comparative study was conducted between a flexible medical device conduit design having a first sharp edge (i.e., sharp head 604 of FIG. 6A) and a commercially available infusion set needle and cannula that requires a "comfortable" or acceptable level of insertion force for placement into the skin.

The method of testing comprised inserting flexible medical device conduits having varying tip angles into Monmouth rubber, a skin phantom material, on top of foam using an Instron machine at a rate of 10 millimeters per minute. The insertion force for different tip angles is presented in Table 1.

Table 1: insertion Force as a Function of Tip Angle

| Device | Tip Angle | Insertion Force (grams) | Standard Deviation (grams) |
| --- | --- | --- | --- |
| Conduit 1 | 43 | 154 | 9 |
| Conduit 2 | 31 | 133 | 4 |
| Conduit 3 | 20 | 118 | 4 |
| Reference | NA | 116 | 10 |

The data in Table 1 indicate that insertion force reduces as a function of tip angle. Tip angle A, therefore, generally ranges from about 20 degrees to about 43 degrees and is typically about 20 degrees.

FIG. 7 is a simplified cross-sectional depiction of an elongated strip 702 with two channels 712a and 712b as can be employed in embodiments of the present invention. Elongated strip 702 has an H-shaped cross-section. When combined with a flexible tube (not shown in FIG. 7), two conduits will be created, thus providing a flexible medical device conduit with conduit operational redundancy or the ability to provide for delivery of two different fluids in each of the conduits (for example, insulin and Smylin). Moreover, an H-shaped cross-section provides additional structural flexibility in comparison to a C-shaped cross-section as depicted in, for example, FIG. 8 described below. Each channel 712a and 712b is, for example, about 0.001 inch to about 0.003 inches, and typically about 0.002 inches, in height (labeled as H in FIG. 7). The thickness of the cross-bar portion (labeled as C in FIG. 7) is generally about 0.0005 inches to about 0.002 inches and is typically 0.001 inch.

FIG. 8 is a simplified cross-sectional depiction of another elongated strip 802 with channel 812 therein as can be employed in embodiments of the present invention. The cross-section of elongated strip 802 is C-shaped and channel 812 includes a base portion 814 with two walls 816. The thickness of base portion 814 (labeled as B in FIG. 8) is generally about 0.0005 inches to about 0.003 inches and is typically about 0.001 inch. The height of each wall 816 (labeled as W in FIG. 8) is generally about 0.003 inches to about 0.006 inches and is typically about 0.004 inches.

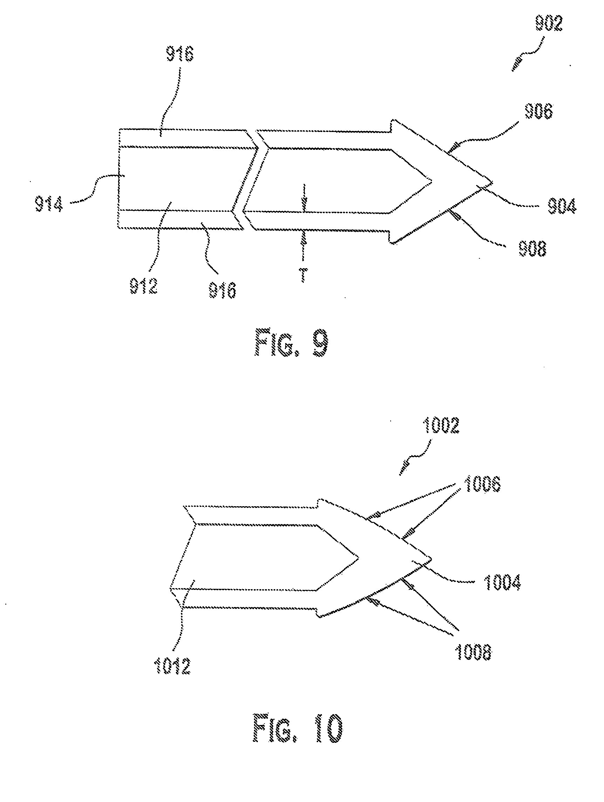

FIG. 9 is a simplified depiction of another elongated strip 902 with channel 912 and sharp head 904 as can be employed in embodiments of the present invention. Sharp head 904 includes a first edge 906 and a second edge 908, both of which are sharp. The cross-section of elongated strip 902 is C shaped and channel 912 includes a base portion 914 with two walls 916. The thickness T of each wall 916 is generally about 0.002 inches to about 0.004 inches and is typically 0.003 inches.

FIG. 10 is a simplified depiction of yet another elongated strip 1002 with a sharp head 1004 and a channel 1012 as can be employed in embodiments of the present invention. Sharp head 1004 includes a first edge 1006 and a second edge 1008, both of which are sharp.

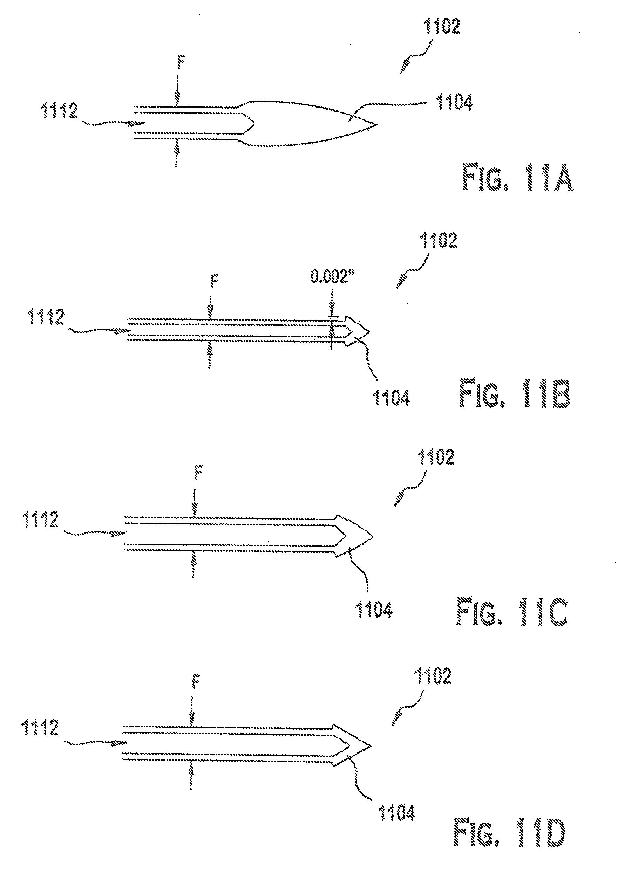

FIGs. 1 1A-11D are simplified depictions of other elongated strips 1102 with sharp heads 1104 and channels 1112 as can be employed in embodiments of the present invention. As shown in FIGs. 11A-11D, the width of the body portion of the elongated strips (labeled as F in the FIGs.) can, for example, be in the range of from about 0.010 inches to about 0.020 inches.

Methods for manufacturing a flexible medical device conduit according to embodiments of the present invention include etching a channel into an elongated Nitinol strip and forming a sharp head on a distal end of the elongated Nitinol strip. The methods also include subsequently jacketing the elongated strip with a flexible tube such that the flexible tube and channel of elongated Nitinol strip define a conduit. Alternatively, stamping and/or coining techniques can be employed to form the channel and sharp head of embodiments of the current invention.

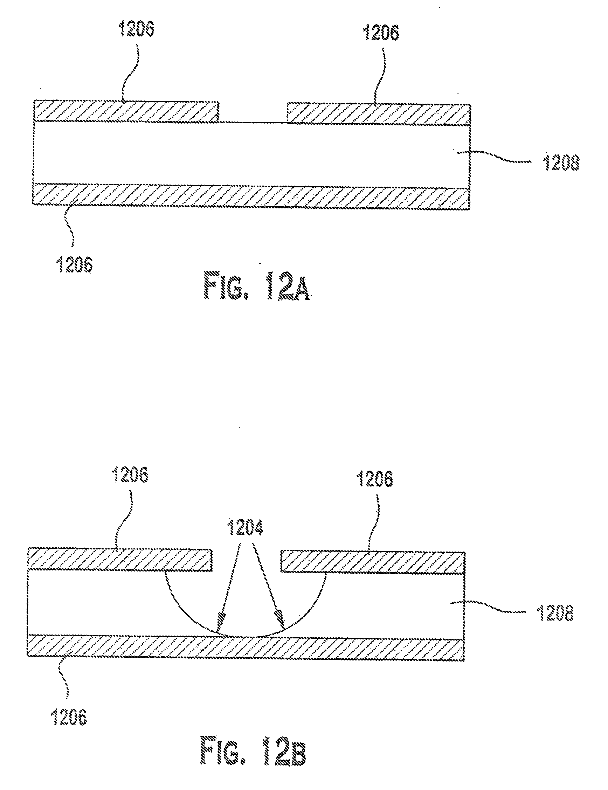

FIGs. 12A and 12B are simplified depictions of stages in an isotropic etching process as can be employed to manufacture sharp heads 1204 of flexible medical device conduits according to the present invention. Sharp heads employed in embodiments of the present invention can be formed using, for example, any suitable etching technique known to those skilled in the art including isotropic chemical etching techniques. Isotropic etching employs a masking layer 1206 disposed on and under a flat sheet 1208 of flexible material, for example, Nitinol. Isotropic etching results in an undercutting of the masking layer 1206 (see FIG. 12B), producing sidewalls with a semi-circular cross-section that creates two sharp heads 1204 at the bottom of the etched surface after the removal of masking layer 1206.

If it is desired to manufacture a curved elongated strip (such as a curved elongated Nitinol strip), curled sheet material can be used instead of the flat sheet depicted in FIGs. 12A and 12B. Alternatively, the strips can be curled in a secondary manufacturing operation following the isotropic etching step.

A channel can be etched on one side of an elongated strip (referred to as a "C" shaped cross section, see FIG. 8B for example) or on both sides (referred to as an "H" shaped cross section, see FIG. 7) of the strip. It is also possible to etch more than one channel on one or both sides of an elongated strip. Etching more than one channel provides some redundancy, in case one of the channels becomes blocked, or the additional channels could be used to deliver different drugs, such as insulin and Symlin.

The sharp head of an elongated strip can be wider than the remainder of the elongated strip (i.e., the body portion) such that when a flexible tube (for example, a polymer jacket flexible tube) is placed around the elongated strip to define a conduit, the leading edge (distal edge) of the flexible tube is aligned with the shoulders of the sharp head (see, for example, FIG. 1A). This configuration optimizes the frontal profile of the flexible medical device conduit, reducing insertion force and preventing the flexible tube from catching on the ineised insertion point in a user's target site, which can lead to undesirable "accordioning" of the polymer. The shoulders on the sharp head are about 0.001 inches to about 0.004 inches and are typically 0.002 inches in height above the body portion (i.e., Distances D and E in FIG. 6A). The height of the shoulders is, for example, typically equal to the thickness of the flexible tube.

The etched channel can extend into the sharp head to provide a conduit opening beyond the flexible tube for fluid to readily flow into the user's target site. Positioning a conduit opening on the side of the sharp head beneficially reduces the chance of blocking the conduit due to coring of target site tissue during insertion.

Many sharp head configurations (see FIGs. 11A through 11D) can be readily manufactured since etching allows for the shape of the sharp head to be created independently on the body portion. In this regard, it should be noted that sharp head configurations with a single sharp edge can be more easily and inexpensively created in comparison to sharp heads with two sharp edges. For example, providing a sharp tip at the intersection of two edges of the sharp head can be more easily accomplished with one sharp edge than with two sharp edges.

A flexible medical device conduit according to embodiments of the present invention can be formed, for example, from an etched elongated Nitinol strip (with a sharp head) with a hear shrink poly(tetrafluoroethylene) or PTFE polymer jacket serving as a flexible tube. Such a PTFE polymer jacket generally exhibits a recovered internal diameter ranging from about 0.007 inches to about 0.015 inches, typically 0.007 inches maximum, a recovered wall thickness ranging from about 0.001 inches to about 0.003 inches, typically 0.002 inches, and an expanded internal diameter ranging from about 0.026 inches to about 0.050 inches, typically 0.026"). Such a heat shrink PTFE tubing will taper down at the juncture with the sharp head, which will facilitate insertion into a user's target site.

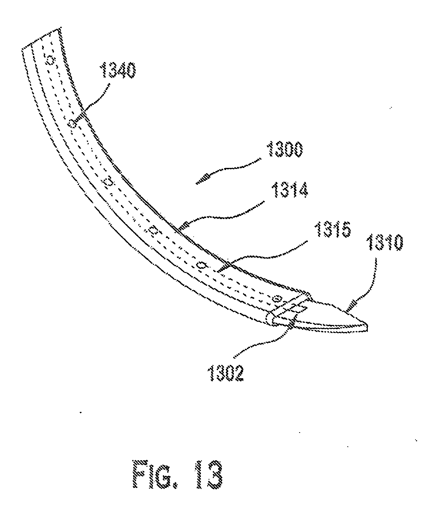

FIG. 13 illustrates a flexible medical device conduit 1300 having an elongated strip 1302 formed of Nitinol jacketed by a flexible tube 1314 and having a sharp head 1310. In this embodiment, holes 1340 are distributed along the length of flexible tube 1314 and allow for fluid to be delivered or extracted from conduit 1315 (defined by elongated strip 1302 and flexible tube 1314) at locations along the length of flexible medical device conduit 1300.

Holes 1340 can be useful for perfusing fluids to a larger area than just near sharp head 1310 of flexible medical device conduit 1300, and also provides for redundancy, reducing the possibility of a total occlusion in the event any of the holes become clogged. For delivery of some drugs such as insulin, if may be desirable to distribute the drug over a broader area to reduce the chances for any localized tissue reaction to the drug, and may help with uptake of the drug by the body. Although holes 1340 are uniformly distributed in FIG. 13, they could be concentrated at the tip, unevenly distributed, or of varying sizes along the length to vary delivery to different areas. In addition, it may be desirable to not provide a conduit opening at the distal end of flexible medical device conduit 1300 near sharp head 1310 such that all fluid exits the conduit of flexible medical device conduit 1300 by way of holes 1340.

Flexible conduit insertion medical devices include a flexible medical cannula according to the invention and an insertion mechanism. The flexible medical cannula (also referred to herein simply as a conduit or flexible conduit) includes an elongated framework formed from a flexible material (e.g., Nitinol) with a body portion, sharp head, distal end and proximal end as defined in claim 1. The insertion mechanism is operatively connected to the flexible medical device conduit and configured to insert a portion of the flexible medical device conduit, including at least the sharp head and the opening, into a user's skin target site.

Flexible conduit insertion medical devices provide for the sharp head to be beneficially obscured from view during insertion and for insertion to occur easily and with minimal steps.

The flexible medical device conduit employed in flexible conduit insertion medical devices has been described above (for example, with respect to FIGs. 1A through 11D and FIG. 13). Exemplary embodiments of insertion mechanisms employed in flexible conduit insertion devices are described below. In this respect it should be noted that the flexible medical device conduit is integrated with the insertion mechanism in that the flexible medical device conduit is not removed, separated or discarded from the insertion mechanism during patient use.

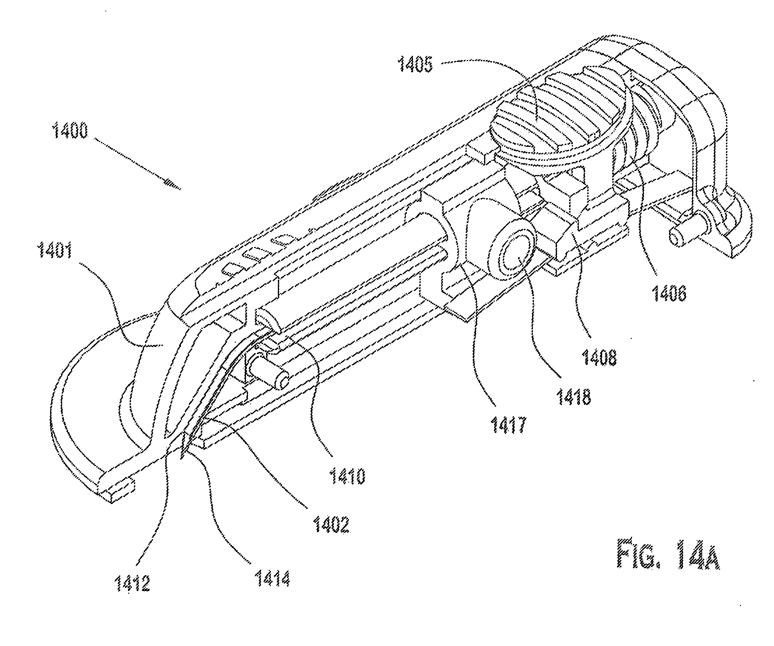

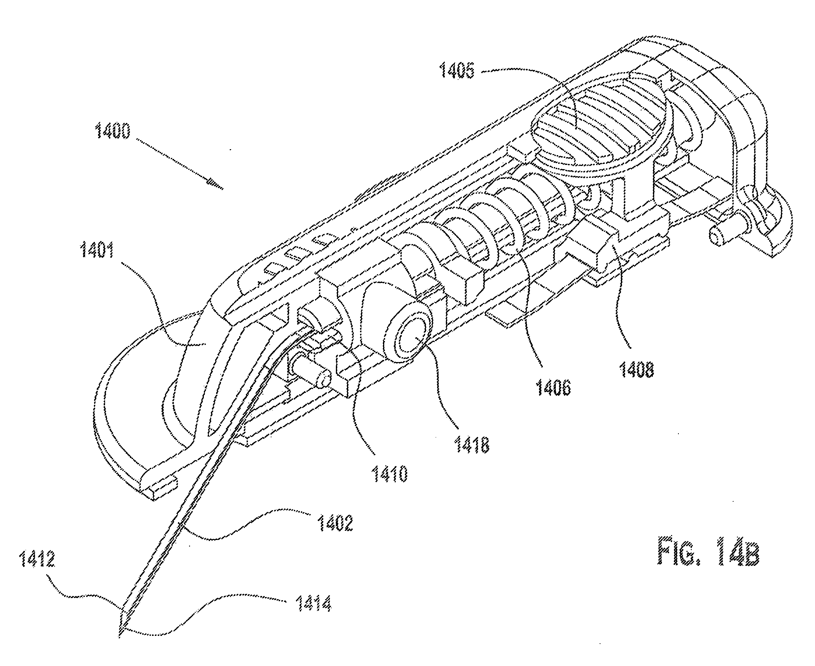

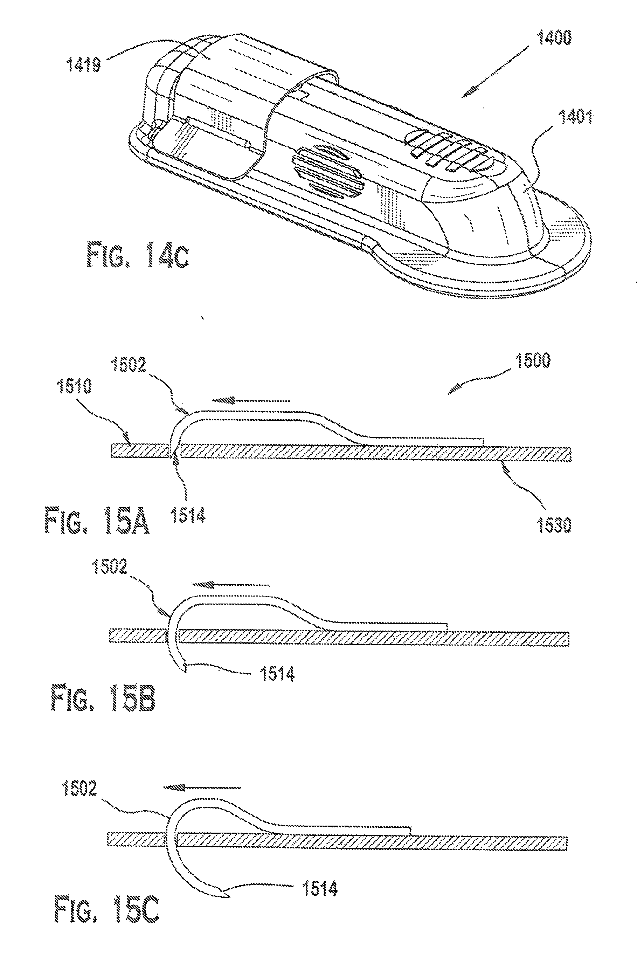

FIGs. 14A and 14B show simplified depictions of a flexible conduit insertion medical device 1400 before deployment of an integrated flexible medical device conduit 1402 and after deployment, respectively. For simplicity, FIGs. 14A and 14B do not depict a skin target site. FIG. 14C is a simplified perspective view of flexible conduit insertion medical device 1400.

Referring to FIGs. 14A-14C, flexible conduit insertion medical device 1400 includes a housing 1401, flexible medical device conduit 1402 and an insertion mechanism (the components of which are hereafter described). Housing 1401 is configured to obscure flexible medical device conduit 1402 from view during use of flexible conduit insertion medical device 1400 and to shield a user from accidental contact with the sharp head of the flexible medical device conduit.

The insertion mechanism of flexible conduit insertion medical device is operatively connected to the flexible medical device conduit 1402 and is configured to insert a portion of the flexible medical device conduit, including at least the sharp bead and the opening thereof, into a user's skin target site. The insertion mechanism includes a firing release button 1405, a firing spring 1406, a latch 1408, and a guide channel 1410 through which the flexible medical device conduit 1402 moves during use, and a plunger 1417. Flexible medical device conduit 1402 includes a distal end 1412 having a sharp head 1414 and a proximal end engaged with a plunger 1417. Plunger 1417 transports flexible medical device conduit 1402 during insertion and may be connected to, for example, an associated insulin supply source (not shown) through a connector port 1418 of flexible conduit insertion medical device 1400.

Flexible conduit insertion medical device 1400 (also referred to herein simply as a "medical device"), can be activated (i.e., insertion commenced, also referred to as "fired") by a user manually pressing the firing release button 1405 to release the latch 1408, or, alternatively, it could be automatically fired by an electromechanical switch (not shown). Medical device 1400 can be provided to a user spring-loaded as shown in FIG. 14A. The medical device is held in the spring-loaded position by the latch 1408. which can be moved out of the way by a manual pressing of the firing release button 1405. The flexible medical device conduit 1402 resides inside guide channel 1410. The majority of the longitudinal axis of guide channel 1410 is approximately parallel to the surface of the user's skin when medical device 1400 has been adhered to the user's skin, but bends at an approximately 45 degree flexible medical device conduit deployment angle towards the user's skin at the distal end of the flexible medical device conduit 1402. The flexible medical device conduit 1402 is normally straight but follows the 45 degree bend of guide channel 1410 because it is formed of flexible material (e.g, superelastic Nitinol). Other suitable deployment angles such as about 20 degrees to about 90 degrees can also be used.

To use medical device 1400, an adhesive backing (not shown) is removed from an adhesive pad (also not shown) attached to the bottom of housing 1401, and the medical device 1400 is applied (adhered) to the user's skin. Medical device 1400 requires minimal dexterity to handle and is relatively small. Therefore, it is easily applied to any skin target site on a user's body that can be touched by the user, for example the top of the buttocks, back of the arm, side, abdomen, and thigh (back, front, or side).

To deploy (insert) flexible medical device conduit 1402, the user removes a protective cap 1419 of medical device 1400 (shown in FIG. 14C) and presses on firing release button 1405, which releases latch 1408 and allows firing spring 1406 to fire the medical device 1400. Flexible medical device conduit 1402 follows guide channel 1410, travels through the 45 degree bend of guide channel 1410, and inserts across the user's skin in, for example, a subcutaneous insertion. The user can press firing release button 1405 with one or more finger, the thumb, the palm, or any part of the hand or arm that is convenient. Very limited dexterity or force is required to activate the insertion mechanism. Alternatively, an electromechanical mechanism can be used to automatically fire the medical device, eliminating the need for the user to activate a release button.

The embodiment of FIGs. 14A - 14C provides for the insertion device to operate by an essentially horizontal movement of the spring and plunger, as well as a large portion of the flexible medical device conduit. Such movement is also referred to is horizontal launching.

Another embodiment of a flexible conduit insertion medical device 1500 (also) referred to simply as "medical device" 1500) in which a flexible medical cannula 1502 is launched in a horizontal direction is illustrated, in a simplified manner, in FIGs. 15A - 15C. FIG. 15A shows medical device 1500 in an initial state, FIG. 15B shows medical device 1500 in an intermediate launching state, and FIG. 15C shows medical device 1500 in a deployed state. Medical device 1500 includes flexible Nitinol conduit 1502 with a sharp head 1514, and a conduit guide 1510 with a surface 1530 representing the interface between a skin target site (not shown) and an adhesive patch of medical device 1500 (also not shown).

Using conventional techniques for forming superelastic Nitinol into curved shapes, flexible medical device conduit 1502 formed from Nitinol is manufactured to have a curved distal end under a no-load condition, as depicted shown in FIG. 15C. The curved distal end of flexible medical device conduit 1502 is initially maintained in a straightened state (see FIG. 15A), since it is disposed within conduit guide 1510 and is constrained by conduit guide 1510.

As shown in FIG. 15A, medical device 1500 is placed in contact with the user's skin with flexible medical device conduit 1302 in a retracted position such that sharp head 1514 of flexible medical device conduit 1502 does not touch the skin. Sharp head 1514 is, in this state, at approximately a 90-degree angle to surface 1530. An insertion mechanism (not shown) of medical device 1500 acts to press on flexible medical device conduit 1502 (i.e., apply a horizontal force) approximately at the location, and in the direction, of the arrows in FIG. 15A. FIG. 15B, and FIG. 15C. Once apprised of the present disclosure, one skilled in the art will recognize that an insertion mechanism can be configured to apply a force in other suitable directions, including perpendicular to the target site (i.e., perpendicular to the arrows of FIGs. 15A-15C).

The pressing results in sharp head 1514 piercing the skin target site, and flexible medical device conduit 1502 slides through conduit guide 1510 as it is inserted across the skin target site and into the body. When sharp head 1514 first contacts the skin, only a short section of flexible medical device conduit 1502 protrudes from conduit guide 1510. This short section of flexible medical device conduit 1502 functions as a rigid member and does not buckle or deflect since it is supported by conduit guide 1510. Thus, sharp head 1514 is able to easily penetrate the skin target site.

As flexible medical device conduit 1502 deploys out of conduit guide 1510 (FIG. 15B to FIG. 15C), it assumes a predetermined curved shape such that in the fully deployed state, sharp head 1514 is below the skin surface at a predetermined depth. For delivering insulin, the predetermined depth can be, for example, from about 3 millimeters to about 12 millimeters, and is typically at a depth from about 4 millimeters to about 8 millimeters to place the distal end of flexible medical device conduit 1502 in the subcutaneous tissue. The depth may be set to be intra-dermal, in the fatty tissue below the skin, or deeper if desired.

As the deployed length of flexible device conduit 1502 increases, it obtains the mechanical freedom needed to become more and more flexible. When flexible medical device conduit 1502 is fully deployed it is sufficiently flexible such that it is confortable for the user to wear.

Because flexible medical device conduit 1502 is curved, a relatively long section resides under the skin, thus preventing it from accidentally coming out of the body. In addition, the process for inserting a flexible medical device conduit described above and further below requires relatively few steps and does not entail removal and discarding of any sharp needles.

After use, the flexible medical device conduit may be removed from the target site by peeling the adhesive from the skin target site and pulling the medical device off of the skin.

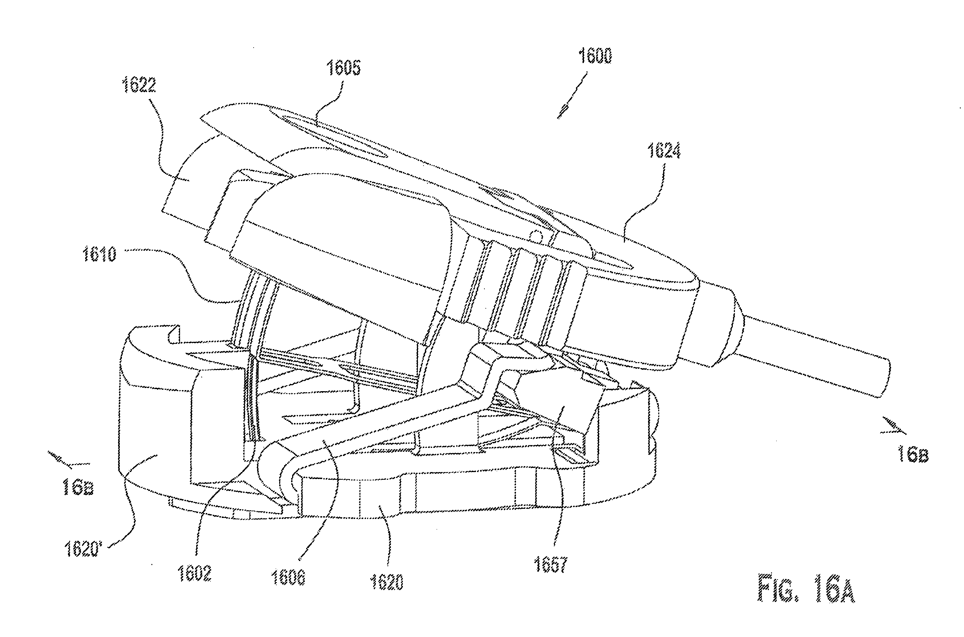

FIGs. 16A - 16H are various simplified views of flexible conduit insertion medical device 1600 (also referred to simply as medical device 1600). Medical a device 1600 includes a base member 1620 having a hinge 1621, a top member 1622 engaged by spring 1606, a flexible medical device conduit 1602 and an automated insertion mechanism (components of which are described below).

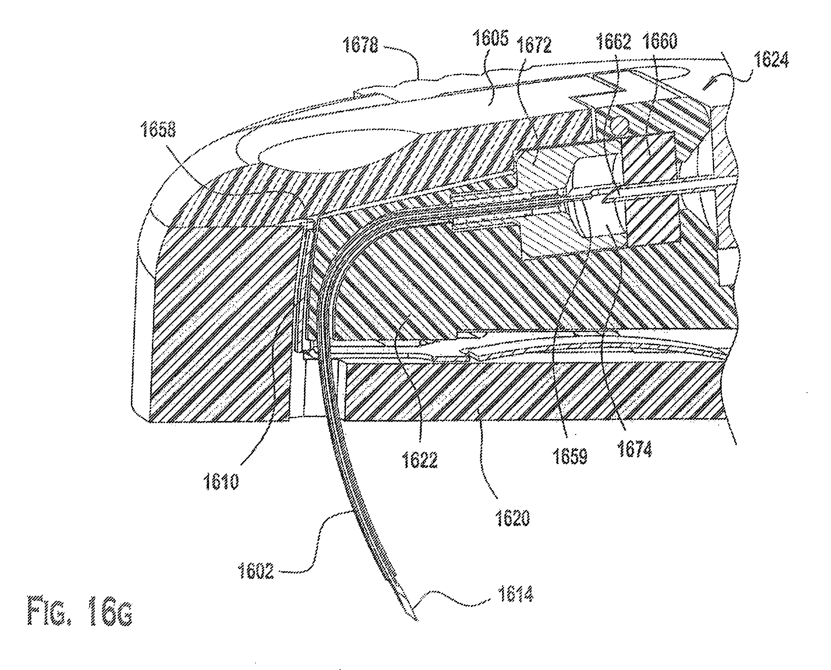

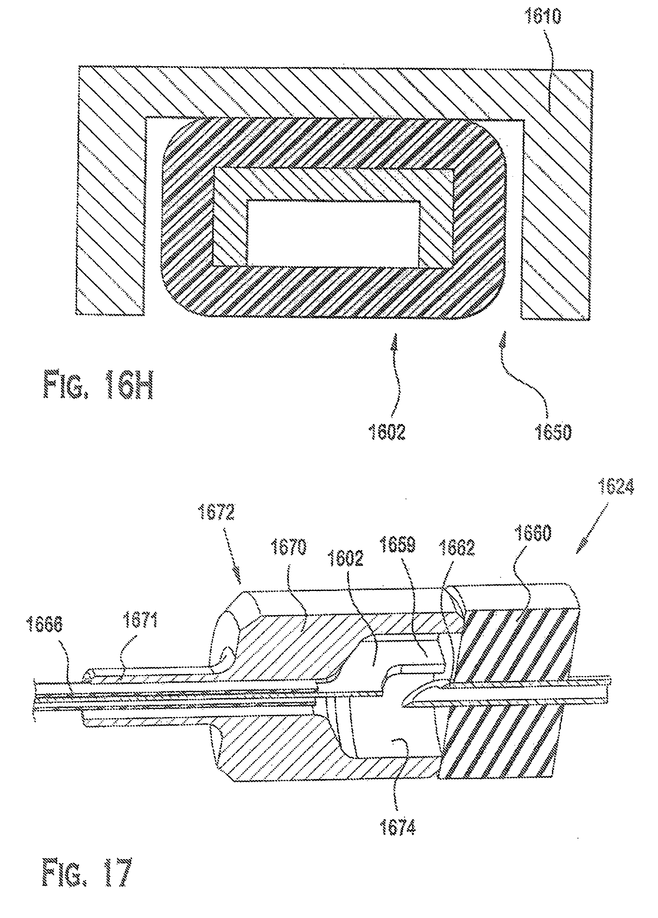

The insertion mechanism of medical device 1600 includes a firing release button 1605, a spring 1606 and a conduit guide 1610 through which flexible medical device conduit 1602 moves. Medical device 1600 is connected to an insulin supply (not shown) by a connector 1624 of medical device 1600 which will be described in more detail below with reference to FIGs. 16G and 17.

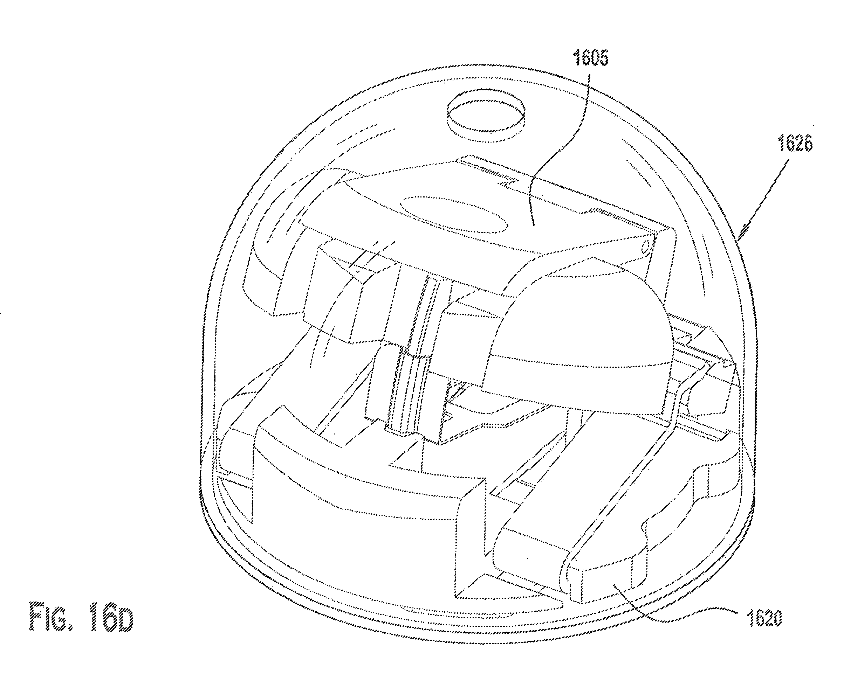

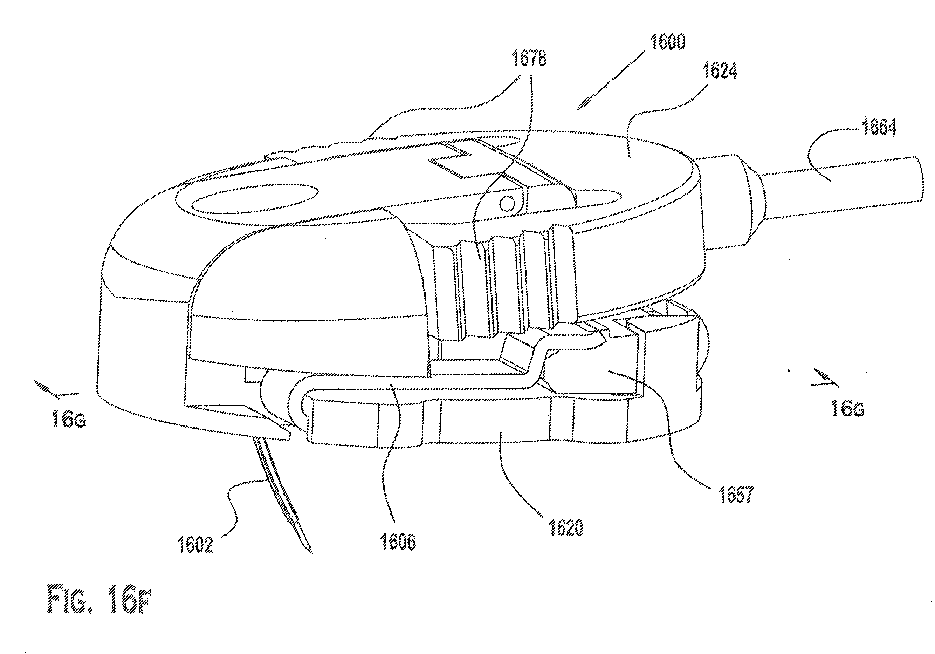

Flexible medical device conduit 1602 includes a distal end 1612 having a sharp head 1614 and a proximal and 1616 engaged by connector 1624 of top member 622. As depicted in FIG. 16D, medical device 1600 may also include a removable protective cover 1626 that serves as a handle for placing the device on the skin and prevents accident deployment of the device. Protective cover 1626 may optionally include features such as detents to aide in gripping and removing the cover. FIGs. 16A - 16E depict medical device 1600 prior to deployment (insertion) of flexible medical device conduit 1602 into a user's target site (not shown). FIGs. 16F - 16G depict medical device 1600 after deployment. FIG. 16H is a simplified cross-sectional depiction of flexible medical device conduit 1602 cooperating with conduit guide 1610.

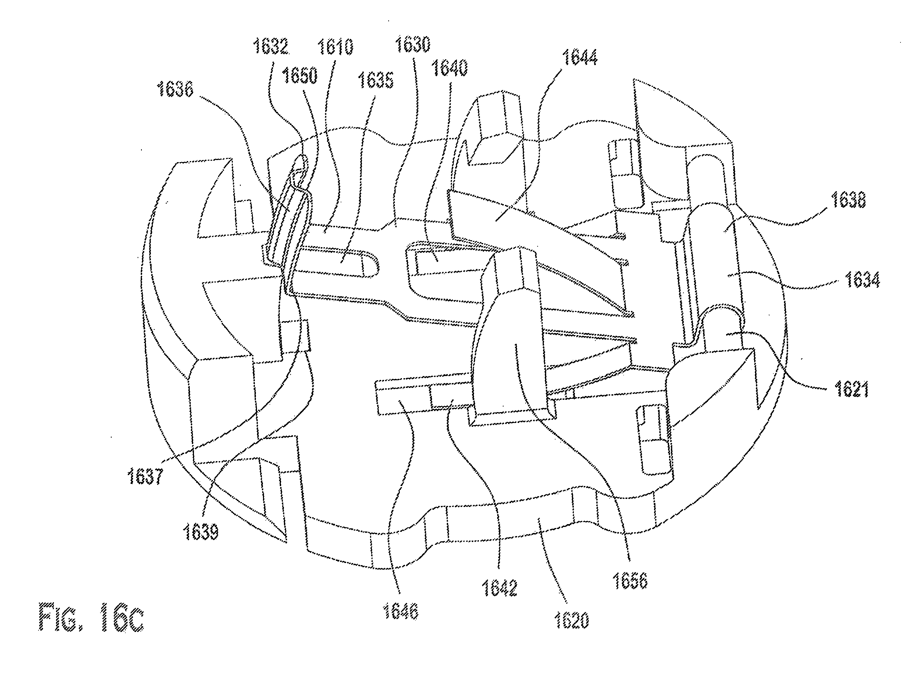

Referring to FIG. 16C, medical device 1600 includes conduit guide 1610 configured to prevent flexible medical device conduit 1602 from buckling during insertion into a user's target site. In addition, a portion (1620' see FIG. 16A) of base member 1620 can, if desired, be configured to serve as an anti-buckling conduit guide. The configuration of medical device 1600 provides anti-bucking support to flexible medical device conduit 1602 at least partially along the length of the flexible medical device conduit. Conduit guides 1610 includes a body 1630 having a first end 1632, a second end 1634 and an opening 1635 through which flexible medical device conduit 1602 can move. A guide portion 1636 is located at first end 1632 and is formed by placing a bend 1637 in body 1630 approximately perpendicular to the plane of body 1630. Guide portion 1636 further includes a channel 1650 configured to operatively cooperate with flexible medical device conduit 1602 as will be described in more detail below.

A hinge portion 1638 of conduit guide 1610 is located at second end 1634. Hinge portion 1638 engages with hinge 1621 of base member 1620. At least one arm projects from body 1630 of conduit guide 1 610 to hold flexible medical device conduit 1602 at a deployment position within medical device 1600. In one exemplary deployment position of flexible medical device conduit 1602, sharp head 1614 is hidden from view within an opening 1639 in base member 1620 and such that the sharp tip does not protrude below the bottom of the device before insertion.

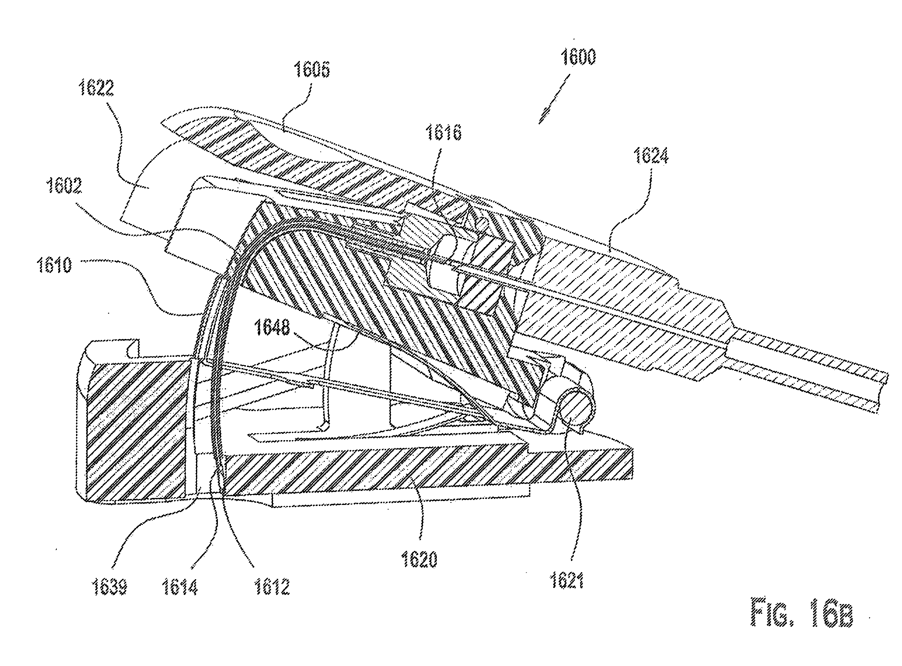

In the embodiment shown in FIGs. 16A - 16G, a first arm 1640 and a second arm 1642 project downward toward base member 1620 and a third arm 1644 projects upward toward top member 1622 of medical device 1600. First arm 1640 and second arm 1642 each engage a recess 1646 in base member 1620 and third arm 1644 engages a lower surface 1648 (shown in FIG. 16B) of top member 1622.

Conduit guide 1610 is formed, for example, of stainless steel or Nitinol and has channel 1650 (or alternatively a groove) in guide portion 1636 configured to operatively cooperate with flexible medical device conduit 1602 (see, for example, FIG. 1 6H). Prior to deployment, flexible medical device conduit 1602 is positioned inside channel 1650 of conduit guide 1610 (see, for example FIGs. 16A and 16B).

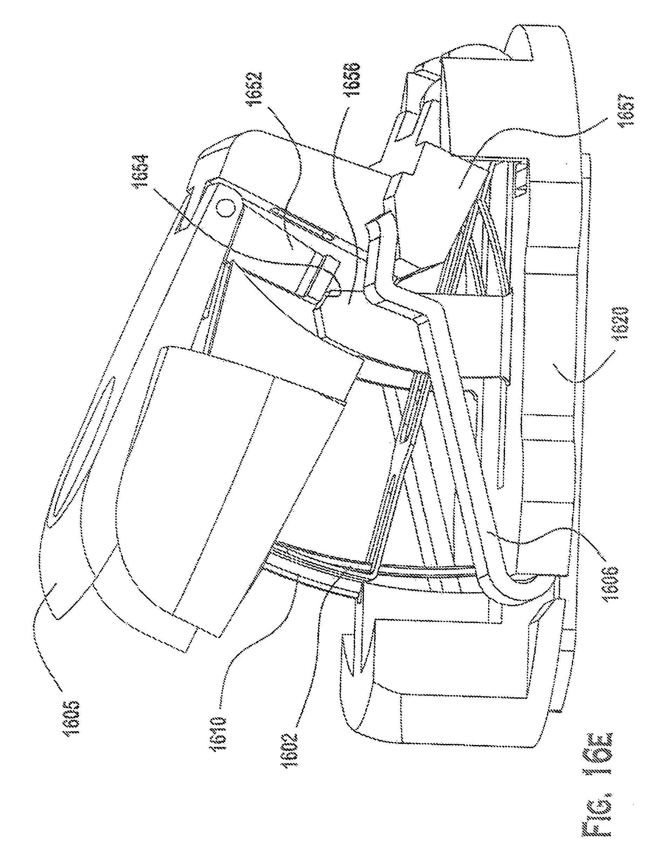

Referring to FIGs. 16A - 16G, to deploy (insert) flexible medical device conduit 1602, after placing the device on the skin, the user removes protective cover 1626 and presses on firing release button 1605, which releases engagement of at least one button arm 1652 (shown in FIG. 16E) with a surface 1654 on at least one projection 1656 projecting from base member 1620. Force on spring 1606 is also released as spring 1606 slides over a protrusion 1657 on top member 1622 causing top member 1622 to rotate about hinge 1621.

The user can deploy medical device 1600 with one or more fingers, the thumb, the palm, or any part of the hand or arm that is convenient. Very limited dexterity or force is required to activate the insertion mechanism. Alternatively, an electromechanical mechanism can be used to automatically fire the device, eliminating the requirement for the user to activate a release button.

When insertion force is applied at the end of flexible medical device conduit 1602 during use (and after medical device 1600 has been adhered to a user by, for example, the use of an adhesive pad on the bottom of the medical device), flexible conduit 1602 bows toward guide portion 1636, pressing against it. Conduit guide 1610 limits the extent to which flexible medical device conduit 1602 bends, thus preventing flexible medical device conduit 1602 from bucking. As the insertion mechanism closes (i.e., automatically transitions from the position of FIG. 16A to the position of FIG. 16F), flexible medical device conduit 1602 pierces the user's skin and enters the subcutaneous tissue (not shown in the FIGs.). Concurrently, conduit guide 1610 folds down into base member 1620 with guide portion 1636 being located in space 1658 between base member 1620 and top member 1622 (see FIG. 16G).

Referring to FIGs. 16G and 17, connector 1624 makes a repeatably sealable liquid connection to flexible medical device conduit 1602 at a proximal end 1659 of flexible medical device conduit 1602, and may make a click that the user may hear and/or feel to alert the user when connector 1624 is fully engaged. For example, as shown in FIG. 16G, top member 1 622 may contain a pierceable septum 1660 and connector 1624 may contain a needle 1662 for piercing septum 1660 to form a liquid connection to flexible medical device conduit 1602. Proximal end 1659 may be wider than the body portion of flexible medical device conduit 1602 and may be Y-shaped to provide clearance for the open end of the needle 1662. Needle 1662 may be connected to a liquid infusion device such as an insulin pump (not shown) via an infusion line 1664 (shown in FIG 16F). Alternatively, a patch pump (not shown) may be removably docked directly onto medical device 1600, eliminating the need for infusion line 1664. One skilled in the art will recognize that connectors employed in embodiments of the present invention can be removably connected or permanently connected to a fluid source, such as an insulin supply, either before or during use.

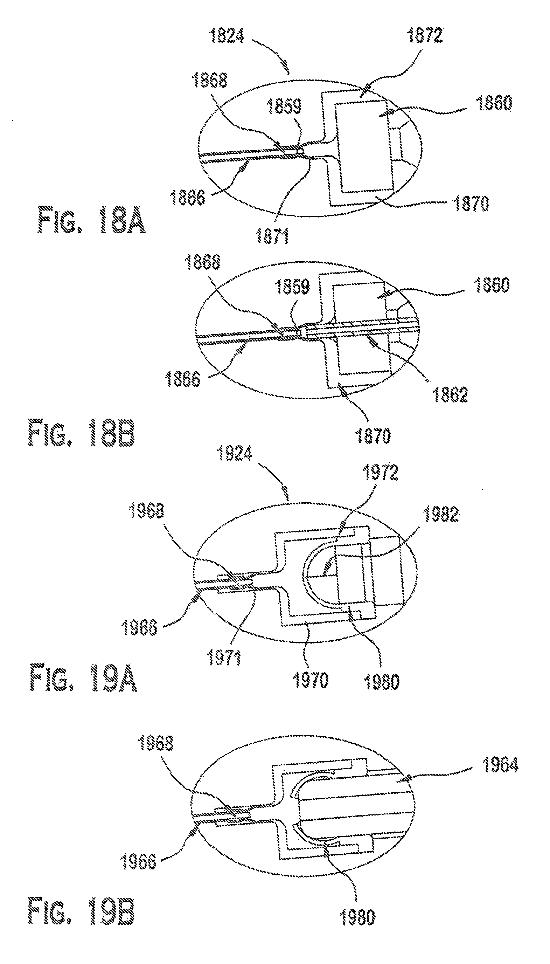

Referring to Fig. 17, connector 1624 includes a cap 1672 that houses proximal end 1659 of flexible medical device conduit 1602. Cap 1672 includes a cylindrically shaped body 1670 having a projection 1671. A tube 1666 (e.g., heat-shrink Teflon tubing) encasing a Nitinol strip 1668 also at least partially surrounds projection 1671 of cap 1672. Tube 1666 creates a sealed lumen 1674 between septum 1660 and Nitinol strip 1668. A girdle 1676 may also be included around tube 1666 to ensure that no fluid leaks occur between flexible medical device conduit 1602 and cap 1672. Girdle 1676 may be formed of, for example, heat-shrink Teflon tubing. This design is simple, straightforward to manufacture, and does not require glue, making it low in cost and reliable. As shown in FIG. 17, body 1670 of cap 1672 abuts septum 1660. In alternative embodiments described below with reference to FIGs. 18A - 18B and 19A - 19B, the cap body at least partially surrounds the septum.

Connector 1624 may be removed from medical device 1600 by depressing two flexible levers 1678 (shown in FIG. 16F) and pulling to disconnect, removing needle 1662 and resealing medical device 1600.

In another embodiment of a connector 1824 shown in FIGs. 18A and 18B, connector 1824 includes a cap 1872 that houses a proximal end 1859 of a conduit. Cap 1872 includes a cylindrically shaped body 1870 having a projection 1871. A tube 1866 encasing a Nitinol strip 1868 also at least partially surrounds projection 1871 to form a seal with cap 1872. Body 1870 of cap 1872 at least partially surrounds a septum 1860, which can be pierced by a needle 1862 connected to an infusion line (not shown).

In yet another embodiment of a connector 1924 shown in FIGs. 19A and 19B, connector 1924 includes a cap 1972 having a cylindrical body 1970 with a projection 1971. A tube 1966 encasing a Nitinol strip 1968 at least partially surrounds projection 1971 to form a seal with cap 1972. Body 1970 houses a valve 1980 that opens when an infusion line 1964 is inserted (FIG. 19B) into connector 1924 and closes when infusion line 1964 is removed, as denoted by a slit 1982 in valve 1980 in FIG. 19A. Alternatively, a ball valve or other type of valve may be used in place of valve 1980.

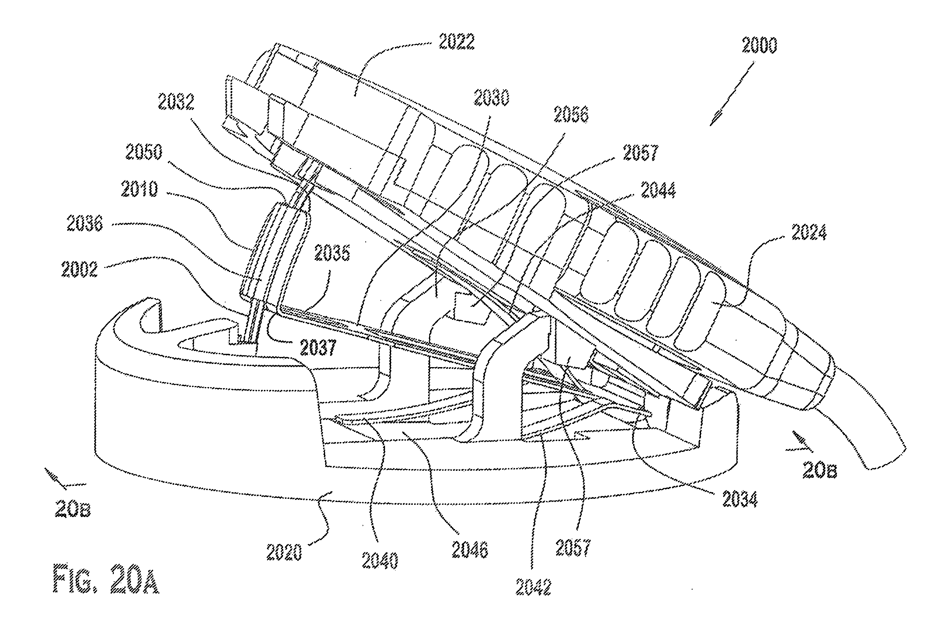

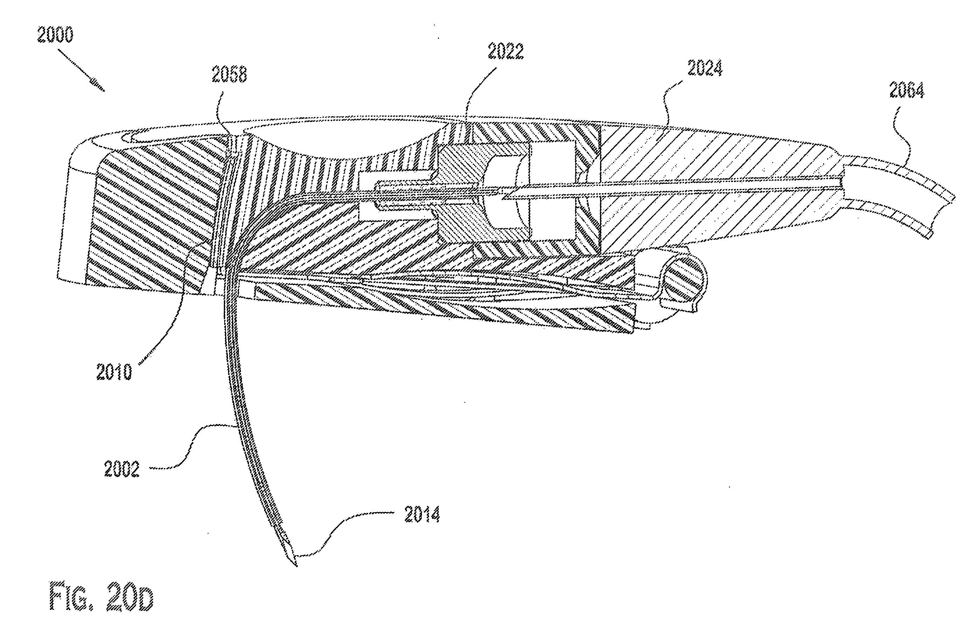

Referring now to FIGs. 20A through 20D, a flexible conduit insertion medical device 2000 (also referred to simply as medical device 2000) is illustrated. Medical device 2000 includes a base member 2020 having a hinge 2021 engaged by a top member 2022 and a conduit guide 2010 through which a flexible medical device conduit 2002 moves. Medical device 2000 is connected to an insulin supply (not shown) by a connector 2024 of medical device 2000. Flexible medical device conduit 2002 includes a sharp head 2014 (see FIG. 20D) on a distal end and is engaged by connector 2024 in top member 2022 (not shown).

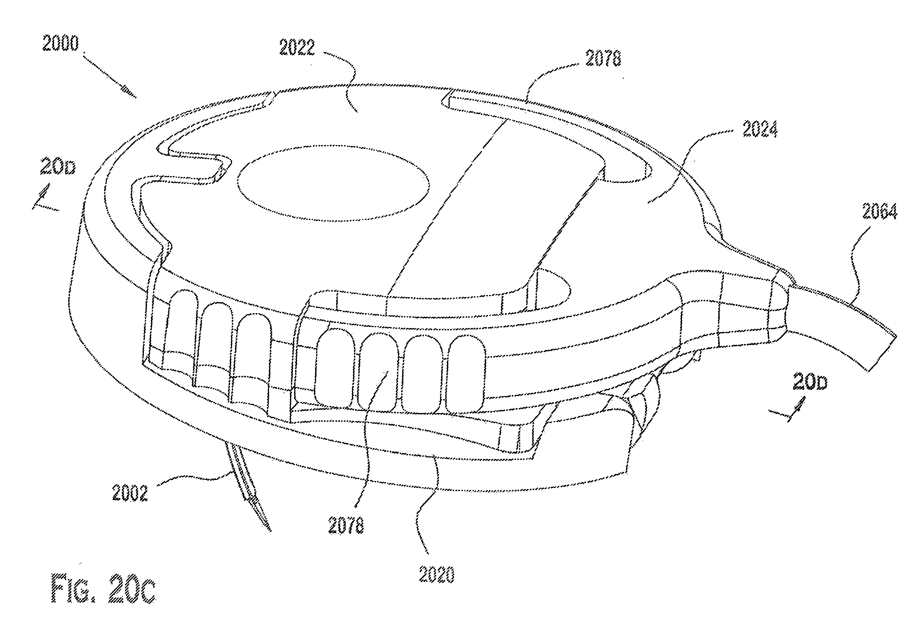

FIGs. 20A - 20B depict medical device 2000 prior to deployment (insertion) of flexible medical device conduit 2002 into a user's target site, FIGs. 20C - 20D depict medical device 2000 after deployment.

Referring to FIG. 20A, medical device 2000 includes conduit guide 2010 to prevent flexible medical device conduit 2002 from buckling during insertion into a user's target site. Conduit guide 2010 collaborates with the member guide in a telescoping manner (compare, for example, FIGs. 20A and 20D). The configuration of medical device 2000 provides anti-buckling support to the flexible medical device conduit 2002 at least partially along its length. Conduit guide 2010 includes a body 2030 having a first end 2032, a second end 2034 and an opening 2035 through which flexible medical device conduit 2002 can move. A guide portion 2036 is located at first end 2032 and is formed by placing a bend 2037 in body 2030 approximately perpendicular to the plane of body 2030. Guide portion 2036 further includes a channel 2050 configured to operatively cooperate with flexible medical device conduit 2002 as is described in more detail below.

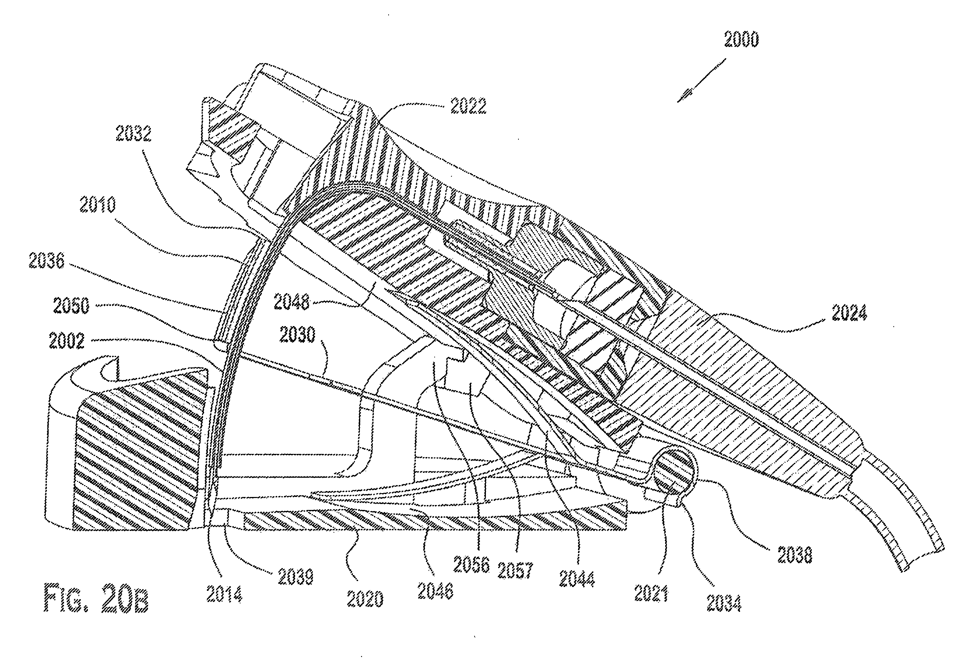

A hinge portion 2038 is located at second end 2034. Hinge portion 2038 engages with hinge 2021 of base member 2020. At least one arm projects from body 2030 of conduit guide 2010 to hold flexible medical device conduit 2002 at a deployment position within medical device 2000. In one exemplary deployment position of flexible medical device conduit 2002, sharp head 2014 is hidden from view within an opening 2039 in base member 2020, and such that the sharp tip of the sharp head does not protrude below the bottom of the device before insertion. In the embodiment shown in FIGs. 20A - 20D, a first arm 2040 and a second arm 2042 project downward toward base member 2020 and a third arm 2044 projects upward toward top member 2022 of medical device 2000. First arm 2040 and second arm 2042 each engage a recess 2046 in base number 2020 and third arm 2044 engages a lower surface 2048 (shown in FIG. 20B) of top member 2022.

Conduit guide 2010 is formed, for example of stainless or Nitinol and has channel 2050 (or alternatively a groove) in guide portion 2036 configured to operatively cooperate with flexible medical device conduit 2002 (see, for example, FIG. 20A). Prior to deployment, flexible medical device conduit 2002 is positioned inside channel 2050 of conduit guide 2010 (see, for example, FIGs. 20A and 20B).

Referring to FIGs. 20A - 20D, to deploy (insert) flexible medical device conduit 2002, the user presses on top member 2022, which causes top member 2022 to rotate about binge 2021 and releases engagement of at least one projection 2056 projecting from base member 2020 with at least one ledge 2057 on top member 2022. Alternatively, the third arm 2044 can be configured to push up on upper portion of device, rotating it upwards until it stops against projections from base member 2020. In this case, to deploy the flexible conduit, it is not necessary to disengage the upper portion from the projections, this providing a smooth operating action. The user can press medical device 2000 with one for more fingers, the thumb, the palm, or any part of the hand or arm that is convenient. Very limited dexterity or force is required to activate the insertion mechanism. Alternatively, an electromechanical mechanism can be used to automatically fire the device, eliminating the requirement for the user top activate a release button.

When insertion force is applied at the end of flexible medical device conduit 2002 during use (and after medical device 2000 has been adhered to a user by, for example, the use of an adhesive pad on the bottom of the medical device), flexible medical device conduit 2002 bows toward guide portion 2036, pressing against it. Conduit guide 2010 (and any guide portion of the base if the device is so configured) limits the extent to which flexible medical device conduit 2002 bends, thus preventing flexible medical device conduit 2002 from buckling. As the insertion mechanism closes (i.e., transitions from the position of FIG. 20A to the position of FIG. 20C via manual user force), flexible medical device conduit 2002 pierces the user's skin and enters the subcutaneous tissue (not shown in the FIGs.). Concurrently, conduit guide 2010 folds down into base member 2020 with guide portion 2036 being located in a space 2058 between base member 2020 and top member 2022. (see FIG. 20D).

After deployment of flexible medical device conduit 2002, connector 2024 can be connected to an insulin supply through an infusion line 2064. Alternatively, an insulin pump (not shown) can be removably docked directly onto medical device 2000, eliminating the need for infusion line 2064. Any suitable repeatable sealable liquid connection such as the embodiments described previously with reference to FIGs. 17 - 19B may be used to connect medical device 2000 to an insulin supply.

Connector 2024 may be removed from medical device 2000 by depressing two flexible levers 2078 (shown in FIG. 20C) and pulling to disconnect.

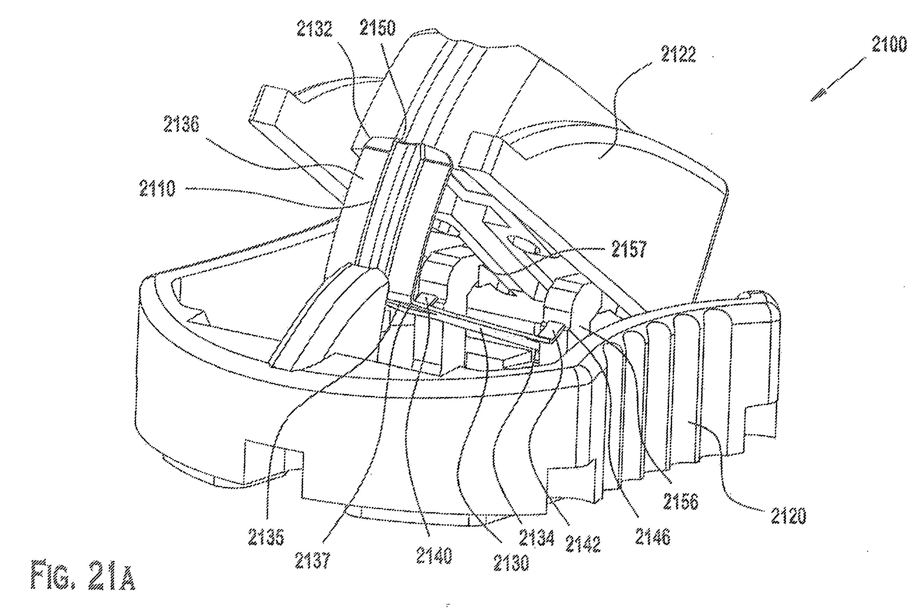

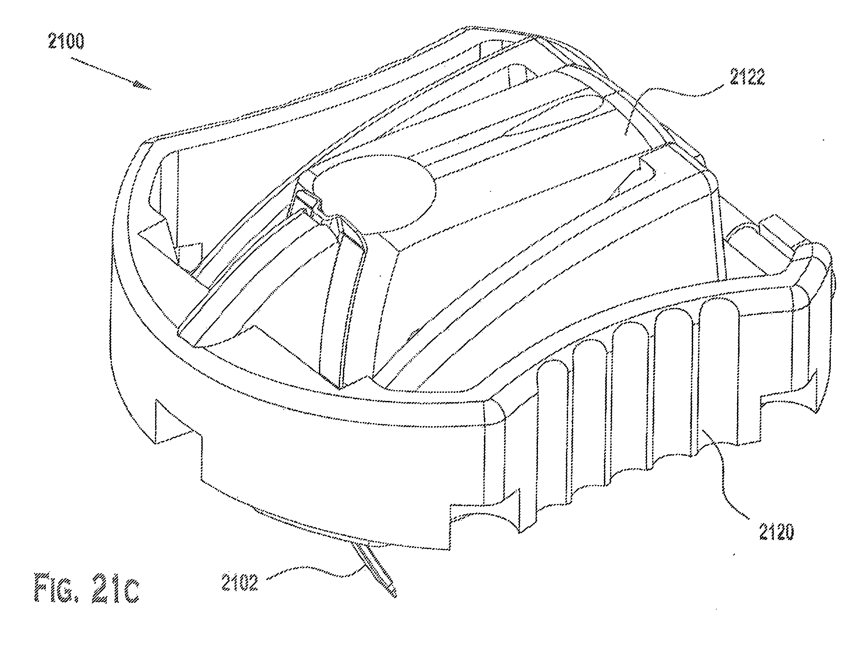

FIGs. 21A - 21D are various simplified views of a flexible conduit insertion medical device 2100 (also referred to simply as "medical device" 2100). Medical device 2100 includes a base member 2120 having a hinge 2121 engaged by a top member 2122 and a conduit guide 2110 through which a flexible conduit 2102 moves. Medical device 2100 is connected to an insulin supply by a connector (not shown). Flexible medical device conduit 2102 includes a sharp head (not shown) on a distal end and is engaged by the connector in top member 2122 (not shown). FIGs. 21A - 21B depict medical device 2100 prior to deployment (insertion) of flexible medical device conduit 2102 into a user's target site. FIG. 21C depicts medical device 2100 after deployment.

Referring to FIG. 21A, medical device 2100 includes conduit guide 2110 to prevent integral flexible medical device conduit 2102 from buckling during insertion into a user's target site. The configuration of medical device 2100 provides anti-buckling support to the flexible medical device conduit 2102 at least partially along its length. Conduit guide 2110 includes a body 2130 having a first end 2132, a second end 2134 and an opening 2135 through which flexible medical device conduit 2102 can move. A guide portion 2136 is located at first end 2132 and is formed by placing a bend 2137 in body 2130 approximately perpendicular to the plane of body 2130. Guide portion 2136 further includes a channel 2150 configured to operatively cooperate with flexible medical device conduit 2102 as will be described in more detail below.

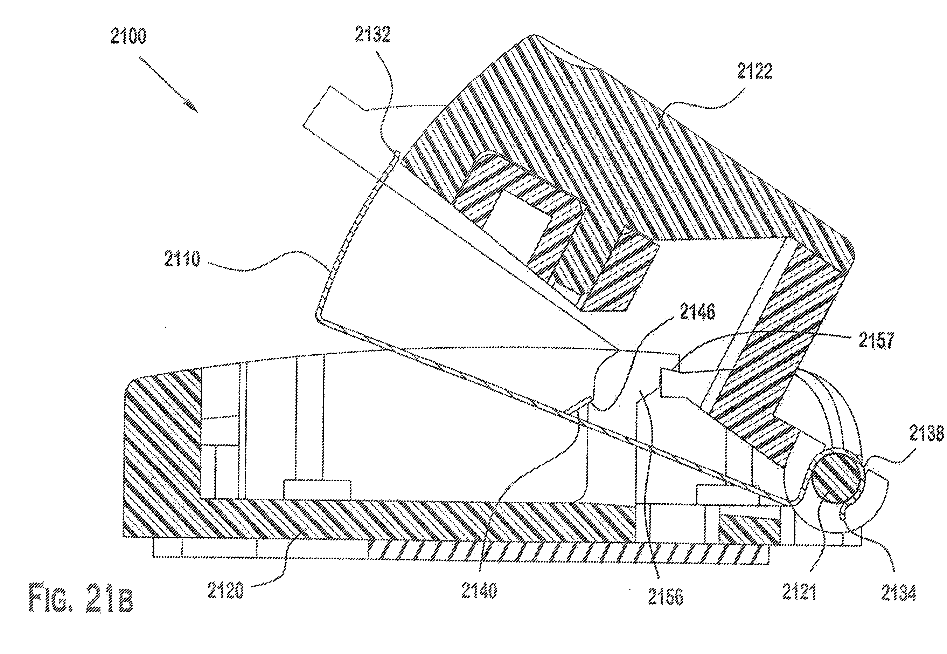

A hinge portion 2138 is located at second end 2134 (see FIG. 21B). Hinge portion 2138 engages with hinge 2121 of base member 2120. At least one finger projects from body 2130 of conduit guide 2110 to hold flexible medical device conduit 2102 at a deployment position within medical device 2000. In one exemplary deployment position of flexible medical device conduit 2102, the sharp head is hidden from view within an opening in base member 2120 (not shown). In the embodiment shown in FIGs. 21A - 21C, a first finger 2140 and a second finger 2142 project upward toward top member 2122 of medical device 2100. Each finger is engaged with a ledge on a projection projecting from base member 2120 (for clarity, FIG. 21B shows only first finger 2140 engaged with a ledge 2146 on a projection 2156).

Conduit guide 2110 is formed, for example, of stainless steel or Nitinol and has channel 2150 (or alternatively a groove) in guide portion 2136 configured to operatively cooperate with flexible medical device conduit 2102. Prior to deployment, flexible medical device conduit 2102 is positioned inside channel 2150 of conduit guide 2110 (see, for example, FIG. 21A and 20B).

Referring to FIGs. 21A - 21D, to deploy (insert) flexible medical device conduit 2102, the user presses on top member 2122, which causes top member 2122 to rotate about hinge 2121 and releases engagement of at least one finger 2140 or 2142 with at least one ledge 2146 of at least one projection 2156 projecting from base member 2020. Concurrently, at least one projection 2156 is released from engagement with at least one ledge 2157 on top member 2122. The user can press medical device 2100 with one or more fingers, the thumb, the palm, or any part of the hand or arm that is convenient. Very limited dexterity or force is required to activate the insertion mechanism. Alternatively, an electromechanical mechanism can be used to automatically fire the device, eliminating the requirement for the user to activate a release button.

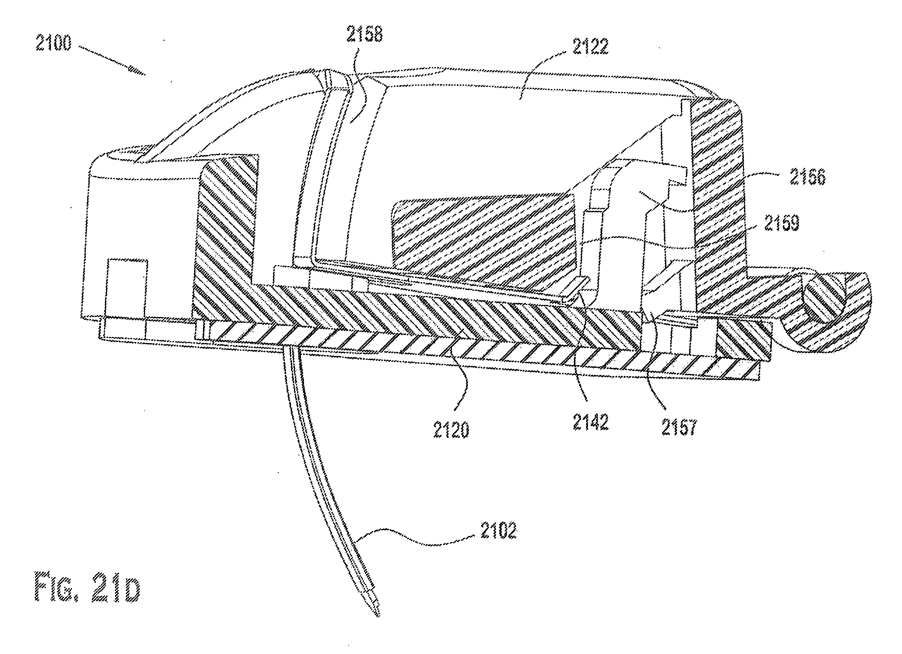

When insertion force is applied at the end of flexible medical device conduit 2102 during use (and after medical device 2100 has been adhered to a oser by, for example, the use of an adhesive pad attached to the bottom of the medical device), flexible medical device conduit 2102 bows toward guide portion 2136, pressing against it. Conduit guide 2110 limits the extent to which flexible medical device conduit 2102 bends, thus preventing flexible medical device conduit 2102 from buckling. As the insertion mechanism closes (i.e., transitions from the position of FIG. 21A to the position of FIG. 21C via manual user force), flexible medical device conduit 2102 pierces the user's skin and enters the subcutaneous tissue (not shown in the FIGs.). Concurrently, conduit guide 2110 rotates down into base member 2120 with guide portion 2136 being located in a first space 2158 between base member 2120 and top member 2122 (see FIG. 21D). First finger 2140 and second finger 2142 each also move into a second space between base member 2120 and top member 2122 (for clarity. FIG. 21D shows only second finger 2142 moving into a second space 2159).

After deployment of flexible medical device conduit 2102, medical device 2100 can be connected to an insulin supply through an infusion line. Any suitable repeatable sealable liquid connection such as the embodiments described previously with reference to FIGs. 17 - 19B may be used to connect medical device 2100 to an insulin supply.

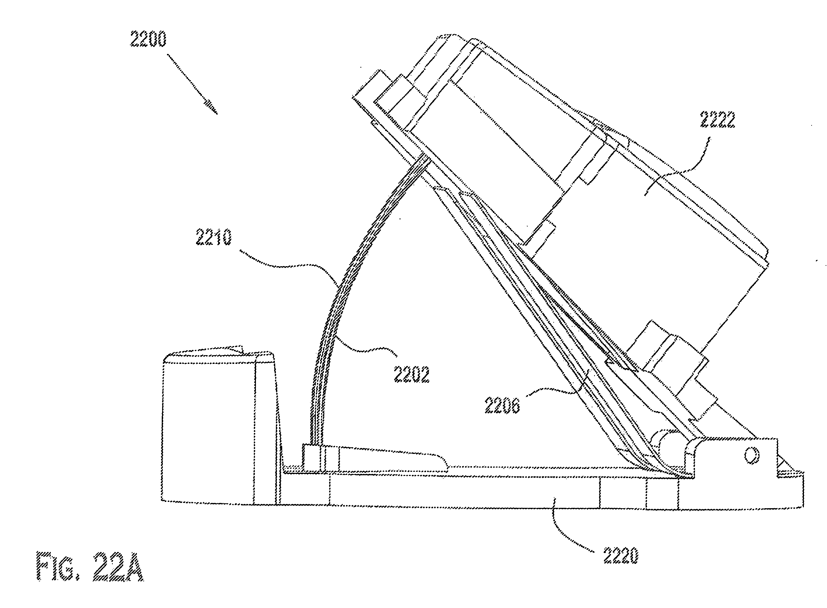

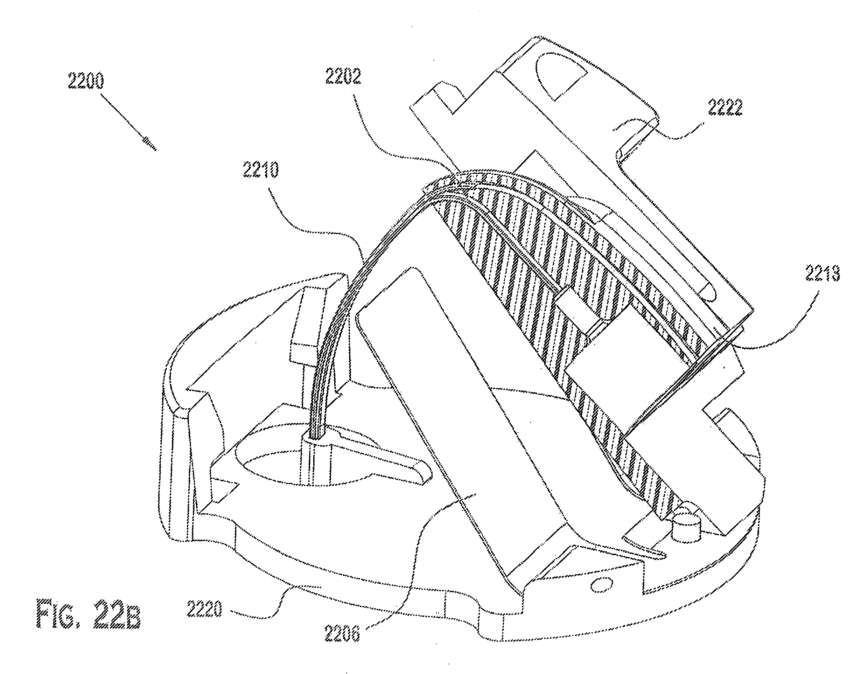

FIGs. 22A - 22B are two simplified views of a flexible conduit insertion medical device 2200 (also referred to simply as a "medical device" 2200) according to another embodiment of the present invention. Medical device 2200 includes a base member 2220, a top member 2222, a conduit guide 2210, an integral flexible medical device conduit 2202 and a leaf spring 2206 that holds integral flexible medial device conduit 2202 in a deployment position (i.e., such that the sharp head of integral flexible medical device conduit 2202 is not visible to the user). FIGs. 22A and 22B depict the medical device prior to deployment (insertion) of integral flexible medical device conduit 2202 into a user's target site.

Conduit guide 2210 prevents integral flexible medical device conduit 2202 from bucking during insertion into a user's target site. The configuration of medical device 2200 provides anti-buckling support to the integral flexible medical device conduit 2202 along its entire length within the medical device.

Conduit guide 2210) is formed, for example, of Nitinol and has at least one channel (or alternatively a groove) configured to operatively cooperate with the flexible medical device conduit. Prior to deployment, integral flexible medical device conduit 2202 is positioned inside the at least one channel of the conduit guide.

When the insertion force is applied at the end of integral flexible medical device conduit 2202 during use (and after medical device 2200 has been adhered to a user by, for example, the use of an adhesive pad attached to the bottom of medical device 2200) integral flexible medical device conduit 2202 bows toward conduit guide 2210, pressing against it. Nitinol conduit guide 2210 limits the extent to which the integral flexible medical device conduit 2202 bends, thus preventing the integral flexible medical device conduit 2202 from buckling. As medical device 2200 closes by manual user force, integral flexible medical device conduit 2202 pierces user's the skin and enters the subcutaneous tissue (not shown in the FIGs.). At the same time, Nitinol conduit guide 2210 travels upwards into a channel 2213 located in top member 2222 and bends (see FiG. 22B). Because Nitinol is superelastic, it bends easily without kinking.

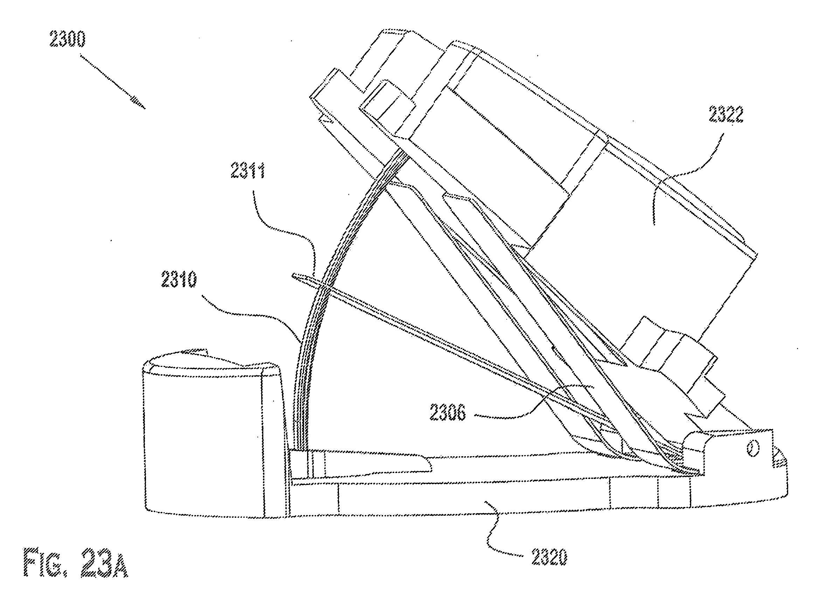

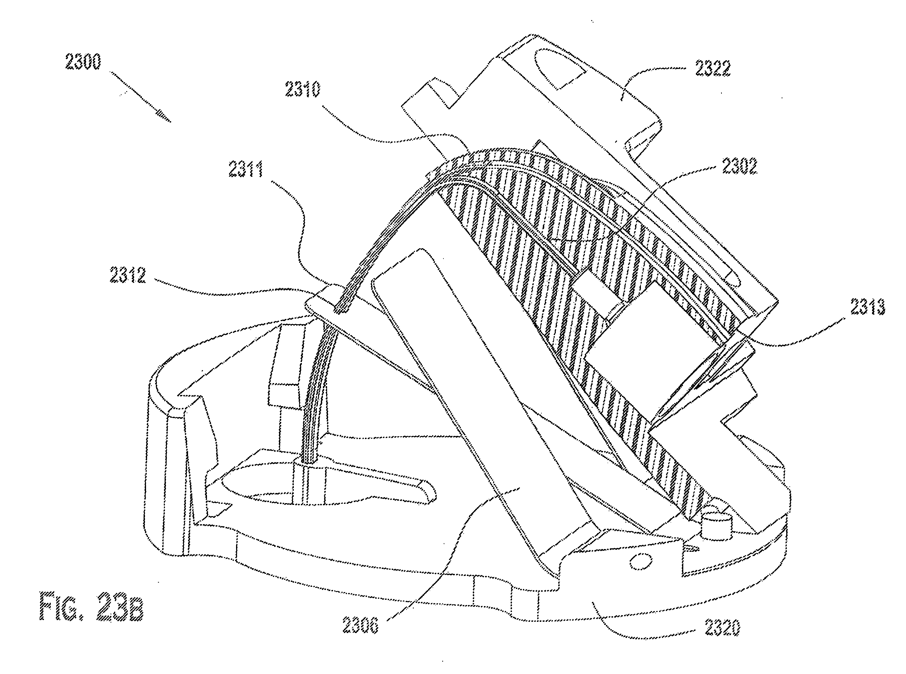

FIGs. 23A - 23B are two simplified views of flexible conduit insertion medical device 2300 (also referred to simply as a medical device). Medical device 2300 includes a base member 2320, a top member 2322, a first conduit guide 2310, a second conduit guide 2311, an integral flexible medical device conduit 2302 and a leaf spring 2306 that holds integral flexible medical device conduit 2302 in a pre-deployment portion (i.e., such that a sharp head of integral flexible medical device conduit 2302 is not visible to the user and does not protrude beyond the opening in the base). FIGs. 23A and 23B depict the medical  device prior to deployment (insertion) of integral flexible medical device conduit 2302 into a user's target site.

First conduit guide 2310 and second conduit guide 2311 prevent integral flexible medical device conduit 2302 from buckling during insertion into a user's target site. First conduit guide 2310 provides anti-buckling support to the integral flexible medical device conduit 2302 along essentially its entire length. Second conduit guide 2311 provides additional anti-buckling support at a location approximately half way between the proximal end and distal end of integral flexible medical device conduit 2302.

First conduit guide 2310 is formed, for example, of Nitinol. Second conduit guide 2311 is formed, for example, of stainless steel. First conduit guide 2310 has a channel (or alternatively a groove) configured to operatively cooperate with integral flexible medical device conduit 2302. Prior to deployment, integral flexible medical device conduit 2302 is positioned inside the channel of first conduit guide 2310. Second conduit guide 2311 includes an aperture 2312 through which integral flexible medical device conduit 2302 moves.

When the insertion force is applied at the end of integral flexible medical device conduit 2302 during use (and after medical device 2300 has been adhered to a user by, for example, the use of an adhesive pad attached to the bottom of medical device 2300), integral flexible medical device conduit 2302 bows toward first conduit guide 2310, pressing against it. The Nitinol first conduit guide 2310 and stainless steel second conduit guide 2311 limit the extent to which integral flexible medical device conduit 2302 bends, thus preventing the integral flexible medical device conduit 2302 from buckling. As medical device 2300 closes by manual user force, integral flexible medical device conduit 2302 pierces user's the skin and enters the subcutaneous tissue (not shown in the FIGs.). At the same time, first conduit guide 2310 travels upwards into a channel 2313 located in top member 2322 and bends (see FIG. 23B). Because Nitinol is superelastic, it bends easily without kinking.

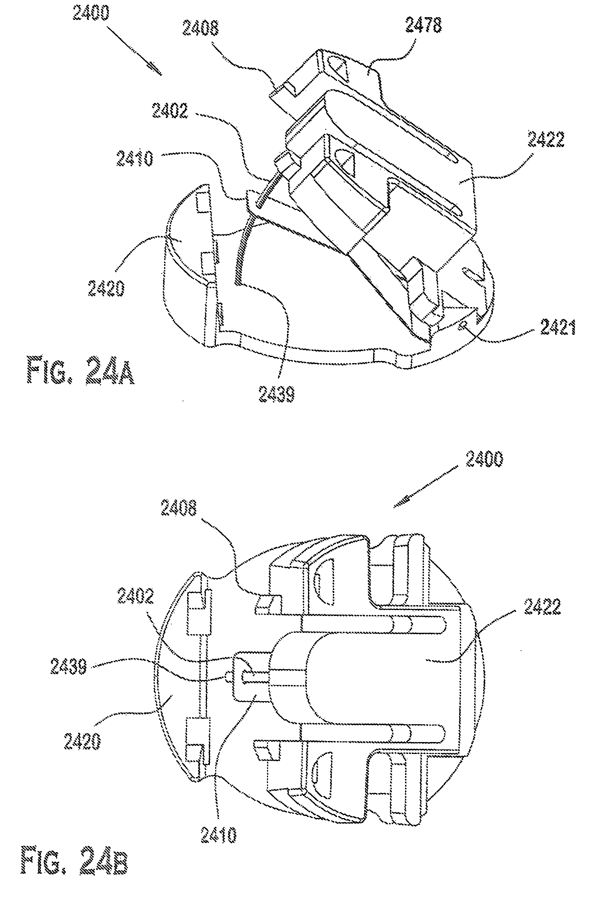

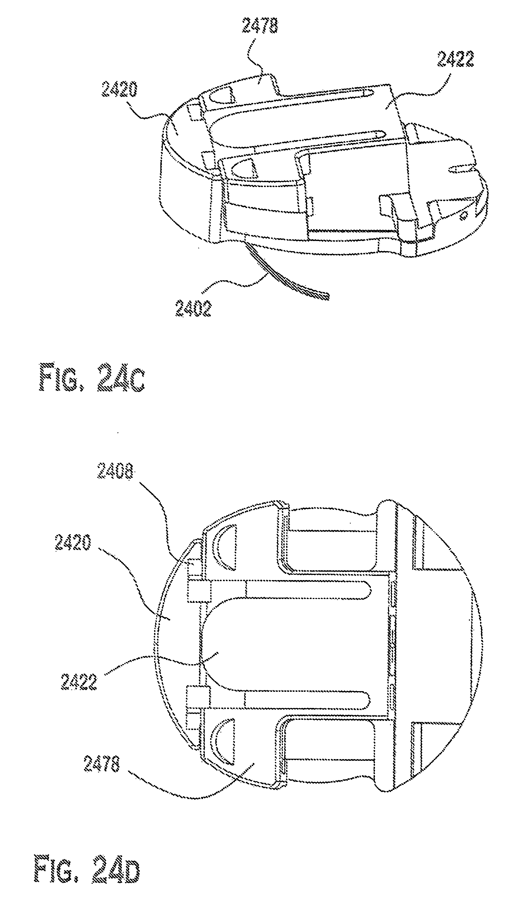

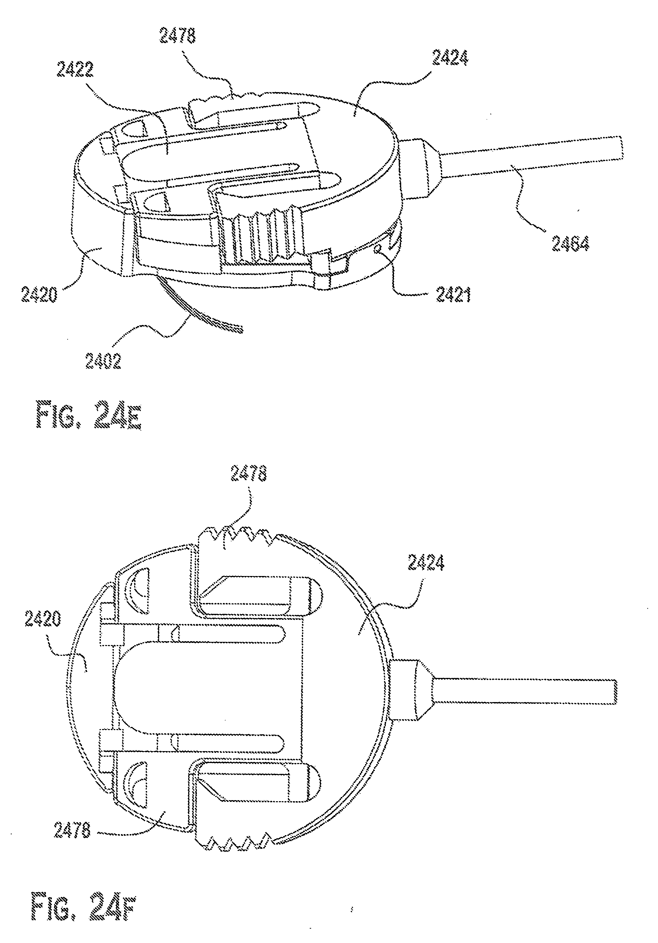

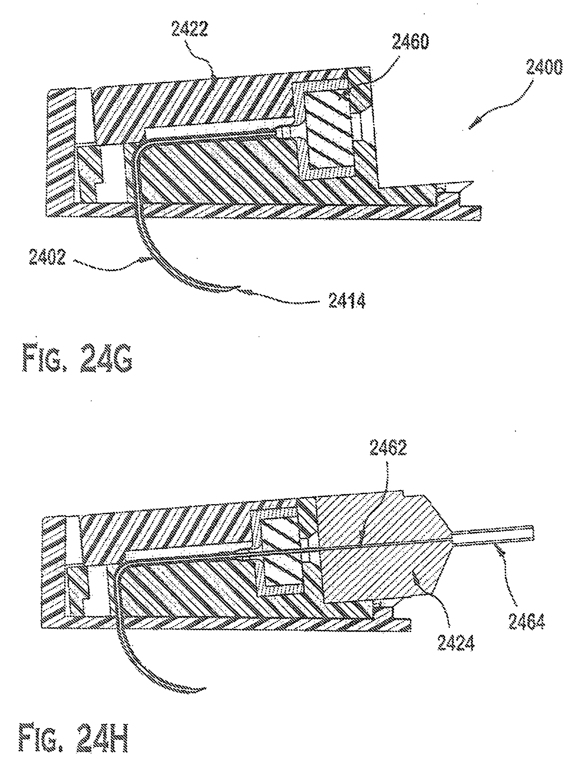

Referring now to FIGs. 24A - 24H, an embodiment of a flexible conduit insertion medical device 2400 (also referred to simply as medical device 2400) incorporating a flexible medical device conduit 2402 is shown. FIGs. 24A and 24B show three dimensional and top views of medical device 2400 in an undeployed state, before flexible medical device conduit 2402 has crossed the skin, and FIG. 24C and 24D show three dimension and top views of medical device 2400 after flexible medical device conduit 2402 has been deployed across the skin. FIGs. 24E and 24F show three dimensional and top views of medical device 2400 after being connected to a liquid infusion device such as an insulin pump. FIGs. 24G and 24H show cross sectional views of medical device 2400 before and after being connected to a liquid infusion device.

Medical device 2400 includes a base member 2420, a top member 2422, a conduit guide 24 10 and a flexible medical device conduit 2402. Base member 2420 has an adhesive pad (not shown) on its underside for attachment to the skin. Flexible medical device conduit 2402 has a sharp head 2414 for piercing the skin, with its opposite end (i.e., proximal end) connected by connector 2424 to septum 2460 housed in top member 2422 (see FIG. 24G).

After attaching medical device 2400 to the skin, the user presses on top member 2422, which rotates about hinge 2421, inserting flexible medical device conduit 2402 into the skin. The center of curvature of flexible medical device conduit 2402 is located approximately at hinge 2421, facilitating deployment of flexible medical device conduit 2402 into the skin. In alternative embodiments, top member 2422 could move in a vertical, horizontal, or angled direction, rather than pivoting.

Conduit guide 2410 prevents flexible medical device conduit 2402 from buckling during the insertion process. It may be desirable to include more than one anti-buckling member. A guide 2439 in base member 2420 guides flexible conduit into the skin during deployment. When flexible medical device conduit 2402 is fully inserted, latch features 2408 lock top member 2422 to base member 2420, preferably making a click that the user may hear and/or feel to alert the user when flexible medical device conduit 2402 is fully deployed.

After deploying flexible medical device conduit 2402, tubing 2464 is attached to medical device 2400 via connector 2424. FIGs. 24C and 24D show three dimensional and top views of medical device 2400 before connecting tubing 2464, and FIGs. 24E and 24F show medical device 2400 after connecting tubing 2464.

Connector 2424 has a means for making a repeatably sealable liquid connection to flexible medical device conduit 2402, at the end opposite sharp head 2414, and preferably makes a click that the user may hear and/or feel to alert the user when connector 2424 is fully engaged. For example, as shown in FIGs. 24G and 24H, the backside of top member 2422 may contain a pierceable septum 2460, and connector 2424 may contain a needle 2462 for piercing septum 2460 to form a liquid connection to flexible medical device conduit 2402 (FIG. 9b and c). Alternatively, backside of top member 2422 may contain a normally closed valve that opens when connector 2424 is attached, and re-closes when connector 2424 is removed. A ball valve or other type of valve may be used instead of the type depicted in FIGs. 24G and 24H.

Connector 2424 may be removed from medical device 2400 by depressing two flexible levers 2478 and pulling to disconnect, removing needle 2462, and resealing medical device 2400.

The opposite end of tubing 2464, not shown, is configured to connect to a liquid infusion device such as an insulin pump. Instead of connecting to a conventional insulin pump via tubing 2464, a patch pump may be removably docked directly onto medical device 2400, eliminating the need for tubing 2464. In the latter case, the outlet of the patch pump would contain needle 2462 or tubing 2464 to form a fluid connection between the pump and medical device 2400.

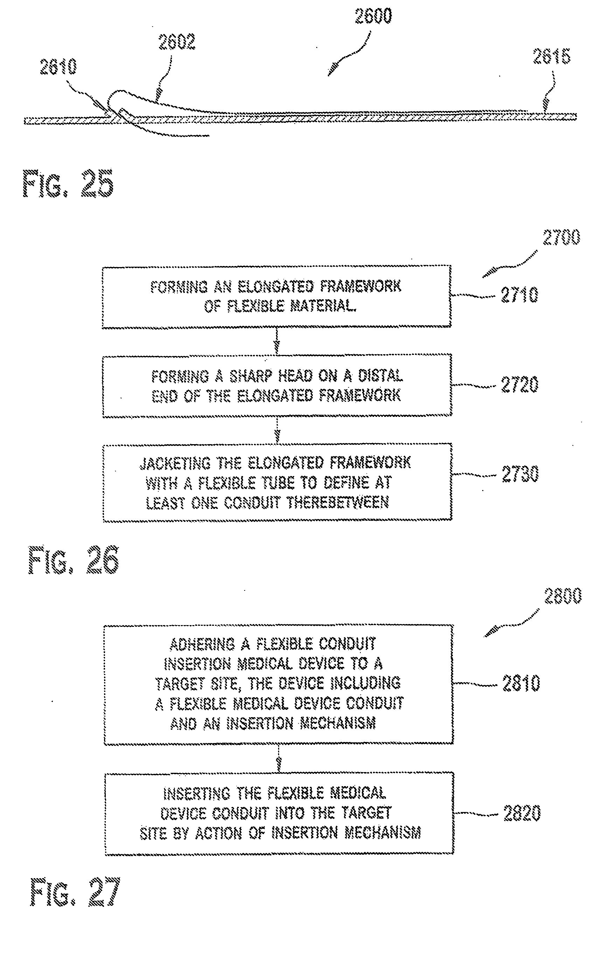

FIG. 25 is a simplified depiction of a flexible conduit insertion medical device 2600 as can be employed in embodiments of the present invention. In FIG. 25, a flexible medical device conduit 2602 formed of Nitinol has a straight section at its end and inserts at of reverse 45-degree angle to skin/device interface 261 5 through conduit guide 2610. allowing for shadow placement of the distal end of the flexible medical device conduit below the skin. The term "reverse" as employed immediately above is with respect to the insertion angle shown in FIG. 14B.

Non-limiting examples of uses in which flexible medical device conduits according to the present invention may be used to include; flexible subcutaneous devices for extracting biological samples such as interstitial fluid or blood for performing analyses such as measuring glucose levels, flexible devices for inserting, positioning, and housing subcutaneous sensors such as glucose sensors; flexible, steerable endoscopes; flexible puncture needles used in interventional radiology for treating slipped disks; flexible, steerable catheters for interventional cardiology applications such as treating chronic total occlusions; flexible, steerable needles for navigating in brain issue; flexible biopsy needles; flexible ureteroscopes; flexible catheter-based needles for transvascular delivery of drugs, cells, and genetic material such as DNA;

and flexible transurethral injection systems. In addition to delivering or extracting liquid or tissue to or from the body, the flexible conduit disclosed here also may be used for deploying devices such as stents, wires, or snares, or for routing wires or optical fibers.

The sharp head of flexible medical device conduits according to embodiments of the present invention remains in the target site during use of the medical device (for example during the administration of insulin) and is only removed, for example, when the entire flexible medical device conduit is removed from the target site. Since the flexible medical device conduit is highly flexible (for example, being formed of Nitinol and a flexible polymer tube), it can remain inserted without undue pain or discomfort during use.

FIG. 26 is a flow diagram depicting stages in a method 2700 for manufacturing a flexible medical device conduit according to an embodiment of the present invention. Method 2700 includes forming an elongated framework of flexible material (e.g., Nitinol) at step 2710, and creating a sharp head on a distal end of the elongated framework using, for example, an isotropic etching, stamping or coming technique (see step 2720).

The method also incudes the step of the elongated framework with a flexible tube such that the fiexible tube and the elongated framework define at least one conduit therebetween, as set forth in step 2720. Furthermore, once apprised of the present disclosure, one skilled in the art will recognize that method 2700 can be readily modified to incorporate any of the manufacturing techniques and to create any of the characteristics and features described herein with respect to flexible medical device conduits and flexible conduit insertion devices according to embodiment of the present invention.

FIG. 27 is a flow diagram depicting stages in a method 2800 for inserting a flexible medical device conduit into a target site according to an embodiment of the present invention.

Method 2800 includes, at step 2810, adhering flexible conduit insertion medical device, with a flexible medical device conduit and an integrated insertion mechanism, to a target site (e.g., a user's skin target site). The flexible medical device conduit thus adhered has been described herein with respect to flexible medical device conduits according to the present invention including, for example, those of FIGs. 1A through 11D and FIG. 13. The insertion mechanism is operatively connected to, and integrated with, the flexible medical device conduit and is configured to insert a portion of the flexible medical device conduit, including at least the sharp head and the opening, into a user's skin target site.

The flexible medical device conduit is partially inserted into the target site by action of the insertion mechanism, as set forth in step 2820. Furthermore, once apprised of the present disclosure, one skilled in the art will recognize that method 2800 can be readily modified to incorporate any of the procedures, uses, methodologies and actions described herein with respect to flexible medical device conduits, flexible conduit insertion medical devices, and methods for manufacturing flexible medical device conduits according to embodiments of the present invention.

A method for manufacturing a flexible medical device conduit, the method comprising: forming an elongated framework of flexible material creating a sharp head on a distal end of the elongated strip; and jacketing the elongated framework with a flexible tube such that the flexible tube and the elongated framework define at least one conduit therebetween.

The method wherein the flexible material is Nitinol. The method wherein the elongated framework is an elongated strip with a longitudinal axis extending from a distal end to the proximal end of the elongated strip; and further including the step of: forming at leasr one channel in the elongated strip, the at least one channel disposed at least partially parallel to the longitudinal axis; and wherein the channel and flexible tube define the at least one conduit. The method wherein the steps of forming the at least one channel and creating a sharp head employ an etching technique. The method wherein the steps of forming the at least one channel and creating a sharp head employ at least one or a stamping technique and a coining techique. The method wherein the step of creating a sharp head employs an isotropic etching technique. The method wherein the step of creating a sharp head employs at least one of a stamping technique and a coining technique. The method wherein the step of creating an elongated framework employs a crimping technique. The method wherein the step of creating an elongated framework employs a at least one of a stamping technique and a coining technique. The method wherein the jacketing step includes the tube with at least one shoulder of the sharp head.

A method for manufacturing a flexible medical device conduit includes forming an elongated framework of flexible material (e.g., Nitinol), and creating a sharp head on a distal end of the elongated strip using, for example, an isotropic etching technique, a stamping technique and/or a coining technique. The method also include the step of jacketing the elongated framework with a flexible tube such that the flexible tube and the elongated framework define at least one conduit therebetween.

A flexible conduit insertion medical device comprising: an elongated framework formed from a flexible material the elongated framework having: a body portion; a sharp head; a distal end; and a proximal end; and a flexible tube at least partially jacketing the elongated framework between the distal end and the proximal end, wherein the sharp head is disposed at the distal end; wherein the elongated framework and flexible tube define at least one conduit therebetween with at least one opening therealong; and wherein the sharp head is configured for target site insertion; and an insertion mechanism operatively connected to, and integrated with, the flexible medical device conduit, the insertion mechanism configured to insert, a portion of the flexible medical device conduit, including at least the sharp head and the opening, into a target site.

The flexible conduit insertion medical device wherein the insertion mechanism configured to subcutaneously insert at portion of the flexible medical device conduit, inciuding at least the sharp head and the opening into a skin target site. The flexible conduit insertion medical device wherein the at least one opening is at the distal end. The flexible conduit insertion medical device, wherein the flexible material is Nitinol. The flexible conduit insertion medical device wherein the distal end of the flexible medical device conduit is formed to have a no load state curved shape. The flexible conduit insertion medical device wherein the insertion mechanism includes a guide channel configured for movement of the flexible medical device conduit therethrough during insertion. The flexible conduit insertion medical device wherein the guide channel is configured to provide a flexible medical device conduit deployment angle of approximately 45 degrees with respect to the target site.

The fiexible conduit insertion medical device wherein the guide channel is configured to provide a flexible medical device conduit deployment angle of approximately 45 reverse degrees with respect to the target site. The flexible conduit insertion medical device wherein the guide channel is further configured to prevent deflection and buckling of the flexible medical device conduit during insertion. The flexible conduit insertion medical device further including a conduit guide configured to prevent deflection and buckling of the flexible medical device conduit during insertion. The flexible conduit insertion medical device further including a connection port. The flexible conduit insertion medical device further including a housing. The flexible conduit insertion medical device further including a connector in liquid communication with the proximal end of the flexible medical device conduit. The flexible conduit insertion medical device further including a conduit guide configured to provide ant-bucking support for the flexible medical device conduit along at least a portion of a length of the flexible medical device conduit.

A flexible conduit insertion medical device includes a flexible medical device conduit and an insertion mechanisin. The flexible medical device conduit includes an elongated frame work formned from a flexible material (e.g., Nitinol) with a body portion, sharp ead, distal end and proximal end. The flexible medical device conduit also incudes to flexible tube at least partially jacketing the elongated framework between the distal end and the proximal end. Moreover, the sharp head is disposed at the distal end and is configured for subcutaneous skin insertion and the elongated framework and flexible tube define at least one conduit between the elongated framework and the flexible tube, the conduit having an opening at the distal end. The insertion mechanism is operatively connected to the flexible medical device conduit and configured to insert a portion of the flexible medical device conduit, including at least the sharp head and the opening, into a user's skin target site.

A method for inserting a flexible medical conduit into a user's target site, the method comprising: adhering a flexible conduit insertion medical device to a target site, the flexible conduit insertion medical device including: an elongated framework formed from a flexible material, the elongated framework having: a body portion; a sharp head; a distal end; and a proximal end; and a flexible tube at least partially jacketing the elongated framework between the distal end and the proximal end, wherein the sharp head is disposed at the distal end; wherein the elongated framework and flexible tube define at least one conduit therebetween with at least one opening therealong; and wherein the sharp head is configured for insertion into a target site; and an insertion mechanism operatively connected to, and integrated with, the flexible medical device conduit, the insertion mechanism configured to insert a portion of the flexible medical device conduit, including at least the sharp head and the opening, into the skin target site; and inserting the flexible medical device conduit into the target site by action of the insertion mechanism.

The method wherein the inserting step is a subcutaneous insertion of the flexible medical device conduit and the target site is a user's skin target site. The method wherein the at least one opening is at the distal end. The method further including the step of: connecting the proximal end of the flexible medical device conduit to an insulin supply via a connector of the flexible conduit insertion device.

The method wherein the adhering step includes adhering flexible conduit insertion medical device wherein the flexible medical conduit is permanently connected to an insulin supply. The method wherein the insertion step employs a conduit guide of the flexible conduit insertion device to provide anti-bucking support of the flexible medical device conduit during insertion. The method wherein the flexible material is Nitinol. The method wherein the distal end of the flexible medical device conduit is formed to have a no load state curved shape. The method wherein the insertion mechanism includes a guide channel configured for movement of the flexible medical device conduit therethrough during the insertion step. The method wherein the guide channel is configured to provide a flexible medical device conduit deployment angle of approximately 45 degrees with respect to the target site. The method wherein the guide channel is configured to provide a flexible medical device conduit deployment angle of approximately 45 reverse degrees with respect to the target site. The flexible medical conduit insertion device further including a housing that blocks a user's view of the flexible medical device conduit.

A method for inserting a flexible medical device conduit includes adhering a flexible conduit insertion medical device, with a flexible medical device conduit and an integrated insertion mechanism, to a target site. Moreover, the flexible medical device conduit has an elongated framework formed from a flexible material (e.g., Nitinol) with a body portion, sharp head, distal end and proximal end. The flexible medical device conduit also has a flexible tube at least partially jacketing the elongated framework between the distal end and the proximal end. In addition, the sharp head is disposed at the distal end and is configured for subcutaneous skin insertion and the elongated framework and flexible tube define at least one conduit between the elongated framework and the flexible tube, the conduit having an opening at the distal end. The insertion mechanism is operatively connected to, and integrated with, the flexible medical device conduit, and the insertion mechanism is configured to insert a portion of the flexible medical device conduit, including at least the sharp head and the opening, into a user's skin target site. The method also includes inserting the flexible medical device conduit into the target site by action of the insertion mechanism.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention.

**Claims**

1.  A flexible medical cannula (100, 200, 300, 400, 500, 600) comprising:

    an elongated framework (102, 202, 302, 402, 502, 602, 702, 802, 902, 1002) formed from a flexible material, the elongated framework having:

        a body portion (103);
        a sharp head (110, 604);
        a distal end (104); and
        a proximal end (106); and **characterized by**

    a flexible tube (114, 210, 310, 410, 510) at least partially jacketing the elongated framework between the distal end and the proximal end,
    wherein the sharp head is disposed at the distal end;
    wherein the elongated framework and flexible tube define at least one conduit (115, 215, 315, 415a/b) therebetween with at least one opening (116) therealong for permitting fluid to exit through said conduit; and
    wherein the sharp head is configured for insertion into a target site.

2.  The flexible medical cannula of claim 1 wherein the at least one opening (116) is an opening at the distal end of the at least one conduit.

3.  The flexible medical cannula of claim 1 wherein the sharp head is configured for subcutaneous insertion and the target site is a skin target site.

4.  The flexible medical cannula of claim 1 wherein the elongated framework has a longitudinal axis and a cross-sectional shape that varies in a predetermined manner along the longitudinal axis.

5.  The flexible medical cannula of claim 4 wherein the cross-sectional shape varies along the longitudinal axis such that the distal end is more flexible than the proximal end.

6.  The flexible medical cannula of claim 4 wherein the cross-sectional shape provides equal flexibility in two directions, preferably two orthogonal directions.

7.  The flexible medical cannula of claim 1 wherein:

    the elongated framework is an elongated strip with a longitudinal axis extending from the distal end to the proximal end; and
    wherein the elongated strip has at least one channel formed therein, the at least one channel disposed at least partially parallel to the longitudinal axis; and
    wherein the channel and flexible tube define the at least one conduit.

8.  The flexible medical cannula of claim 7 wherein the at least one channel extends into the sharp head and wherein the at least one conduit defined by the channel and flexible tube has an opening along the sharp head.

9. The flexible medical cannula of claim 1 or claim 8 wherein the flexible material is a superelastic flexible material.

10. The flexible medical cannula of claim 9 wherein the superelastic flexible material is Nitinol.

11. The flexible medical cannula of claim 1 wherein the elongated framework has a C-shaped cross-section, an S-shaped cross-section, a curved cross-section or a rectangular cross-section with an orientation that varies along a length of the flexible medical device conduit.

12. The flexible medical cannula of claim 1 wherein the sharp head has a first sharp edge, a second edge and a tip, with the first edge and second edge defining a tip angle, preferably a tip angle of about 20 degrees.

13. The flexible medical cannula of claim 1 wherein the flexible tube includes a plurality of holes therethrough along a length of the flexible medical device conduit, the plurality of holes being in fluid communication with the at least one conduit.

14. The flexible medical cannula of claim 1 wherein the sharp head has at least one shoulder aligned with the flexible jacket.

15. The flexible medical cannula of claim 1 wherein the flexible tube is tapered at a juncture with the sharp head to, thereby, align with the sharp head.


**Patentansprüche**

1. Flexible medizinische Kanüle (100, 200, 300, 400, 500, 600), die aufweist:

   eine langgestreckte Rahmenanordnung (102, 202, 302, 402, 502, 602, 702, 802, 902, 1002), die aus einem flexiblen Material geformt ist, wobei die langgestreckte Rahmenanordnung aufweist:

   einen Körperabschnitt (103);
   einen scharfen Kopf (110, 604);
   ein distales Ende (104); und
   ein proximales Ende (106); und **gekennzeichnet durch**:

   ein flexibles Rohr (114, 210, 310, 410, 510), das die langgestreckte Rahmenanordnung zwischen dem distalen Ende und dem proximalen Ende mindestens teilweise ummantelt,
   wobei der scharfe Kopf an dem distalen Ende angeordnet ist;
   wobei die langgestreckte Rahmenanordnung und das flexible Rohr mindestens eine Leitung (115, 215, 315, 415a/b) dazwischen definieren mit mindestens einer Öffnung (116) daran, um einem Fluid zu ermöglichen, **durch** die Leitung hindurchzutreten; und
   wobei der scharfe Kopf für einen Einsatz in einen Zielort konfiguriert ist.

2. Flexible medizinische Kanüle nach Anspruch 1, wobei die mindestens eine Öffnung (116) eine Öffnung an dem distalen Ende der mindestens einen Leitung ist.

3. Flexible medizinische Kanüle nach Anspruch 1, wobei der scharfe Kopf für einen subkutanen Einsatz konfiguriert ist und der Zielort ein Zielort der Haut ist.

4. Flexible medizinische Kanüle nach Anspruch 1, wobei die langgestreckte Rahmenanordnung eine Längsachse und eine Querschnittsform aufweist, die in einer vorgegebenen Art entlang der Längsachse variiert.

5. Flexible medizinische Kanüle nach Anspruch 4, wobei die Querschnittsform entlang der Längsachse derart variiert, dass das distale Ende flexibler als das proximale Ende ist.

6. Flexible medizinische Kanüle nach Anspruch 4, wobei die Querschnittsform in zwei Richtungen eine gleiche Flexibilität bereitstellt, vorzugsweise in zwei orthogonalen Richtungen.

7. Flexible medizinische Kanüle nach Anspruch 1, wobei:

die langgestreckte Rahmenanordnung ein langgestreckter Streifen mit einer Längsachse ist, die sich von dem distalen Ende zu dem proximalen Ende erstreckt; und

wobei der langgestreckte Streifen mindestens einen darin geformten Kanal aufweist, wobei der mindestens eine Kanal mindestens teilweise zu der Längsachse parallel angeordnet ist; und

wobei der Kanal und das flexible Rohr die mindestens eine Leitung definieren.

**8.** Flexible medizinische Kanüle nach Anspruch 7, wobei der mindestens eine Kanal sich in den scharfen Kopf erstreckt und wobei die mindestens eine Leitung, die durch den Kanal und durch das flexible Rohr definiert ist, eine Öffnung entlang des scharfen Kopfs aufweist.

**9.** Flexible medizinische Kanüle nach Anspruch 1 oder 8, wobei das flexible Material ein superelastisches flexibles Material ist.

**10.** Flexible medizinische Kanüle nach Anspruch 9, wobei das superelastische flexible Material Nitinol ist.

**11.** Flexible medizinische Kanüle nach Anspruch 1, wobei die langgestreckte Rahmenanordnung eine C-förmige Querschnittsform, eine S-förmige Querschnittsform, eine gekrümmte Querschnittsform oder eine rechteckige Querschnittsform mit einer Orientierung aufweist, die entlang einer Länge der flexiblen medizinischen Leitungsvorrichtung variiert.

**12.** Flexible medizinische Kanüle nach Anspruch 1, wobei der scharfe Kopf eine erste scharfe Kante, eine zweite Kante und eine Spitze aufweist, wobei die erste und die zweite Kante einen Kopfkegelwinkel definieren, vorzugsweise einen Kopfkegelwinkel von etwa 20 Grad.

**13.** Flexible medizinische Kanüle nach Anspruch 1, wobei das flexible Rohr entlang einer Länge der flexiblen medizinischen Leitungsvorrichtung mehrere Löcher dort hindurch enthält, wobei die mehreren Löcher in einer Fluidkommunikation mit der mindestens einen Leitung stehen.

**14.** Flexible medizinische Kanüle nach Anspruch 1, wobei der scharfe Kopf mindestens eine Schulter aufweist, die auf die flexible Ummantelung ausgerichtet ist.

**15.** Flexible medizinische Kanüle nach Anspruch 1, wobei das flexible Rohr an einem Verbindungspunkt mit dem scharfen Kopf spitz zulaufend ist, um sich dadurch auf den scharfen Kopf auszurichten.

**Revendications**

**1.** Canule (100, 200, 300, 400, 500, 600) médicale flexible comportant :

un cadre (102, 202, 302, 402, 502, 602, 702, 802, 902, 1002) allongé constitué d'un matériau flexible, le cadre allongé présentant :

une partie (103) tête ;
une tête (110, 604) affûtée ;
une extrémité (104) distale ; et
une extrémité (106) proximale ; et **caractérisée par**

un tube (114, 210, 310, 410, 510) flexible enveloppant au moins partiellement le cadre allongé entre l'extrémité distale et l'extrémité proximale,
dans laquelle la tête affûtée est disposée au niveau de l'extrémité distale ;
dans laquelle le cadre allongé et le tube flexible délimitent au moins un conduit (115, 215, 315, 415a/b) entre eux avec au moins une ouverture (116) le long de ce dernier afin de permettre à un fluide de sortir par ledit conduit ; et
dans laquelle la tête affûtée est configurée pour être introduite dans un site cible.

**2.** Canule médicale flexible selon la revendication 1 dans laquelle ladite au moins une ouverture (116) est une ouverture au niveau de l'extrémité distale dudit au moins un conduit.

**3.** Canule médicale flexible selon la revendication 1 dans laquelle la tête affûtée est configurée pour être introduite par voie sous-cutanée et le site cible est un site cible de la peau.

**4.** Canule médicale flexible selon la revendication 1 dans laquelle le cadre allongé a un axe longitudinal et une forme en coupe qui varie de manière prédéterminée le long de l'axe longitudinal.

**5.** Canule médicale flexible selon la revendication 4 dans laquelle la forme en coupe varie le long de l'axe longitudinal de sorte que l'extrémité distale est plus flexible que l'extrémité proximale.

**6.** Canule médicale flexible selon la revendication 4 dans laquelle la forme en coupe fournit la même flexibilité dans deux directions, de préférence deux directions orthogonales.

**7.** Canule médicale flexible selon la revendication 1 dans laquelle :

le cadre allongé est une bande allongée présentant un axe longitudinal qui s'étend de l'extrémité distale à l'extrémité proximale ; et
dans laquelle la bande allongée présente au moins un canal à l'intérieur de cette dernière, et au moins un canal disposé au moins partiellement parallèlement à l'axe longitudinal ; et
dans laquelle le canal et le tube flexible délimitent ledit au moins un conduit.

**8.** Canule médicale flexible selon la revendication 7 dans laquelle ledit au moins un canal s'étend dans la tête affûtée et dans laquelle ledit au moins un conduit délimité par le canal et le tube flexible présente une ouverture le long de la tête affûtée.

**9.** Canule médicale flexible selon la revendication 1 ou la revendication 8 dans laquelle le matériau flexible est un matériau flexible super-élastique.

**10.** Canule médicale flexible selon la revendication 9 dans laquelle le matériau flexible super-élastique est du Nitinol.

**11.** Canule médicale flexible selon la revendication 1 dans laquelle le cadre allongé a une section transversale en forme de C, une section transversale en forme de S, une section transversale courbe ou une section transversale rectangulaire avec une orientation qui varie sur une longueur du conduit du dispositif médical flexible.

**12.** Canule médicale flexible selon la revendication 1 dans laquelle la tête affûtée présente un premier bord affûté, un deuxième bord affûté et un bout, avec le premier bord et le deuxième bord délimitant un angle de bout, de préférence un angle de bout d'environ 20 degrés.

**13.** Canule médicale flexible selon la revendication 1 dans laquelle le tube flexible comprend une pluralité de trous traversants sur une longueur du conduit du dispositif médical flexible, la pluralité de trous étant en communication fluidique avec ledit au moins un conduit.

**14.** Canule médicale flexible selon la revendication 1 dans laquelle la tête affûtée présente au moins un épaulement aligné avec l'enveloppe flexible.

**15.** Canule médicale flexible selon la revendication 1 dans laquelle le tube flexible est conique au niveau d'une jonction avec la tête affûtée pour être ainsi aligné avec la tête affûtée.

FIG. 1A

115

114

102

**FIG. 1B**

115    114    116

110

103    102

114

**FIG. 1C**

FIG. 2

FIG. 3

FIG. 4

510

500

502

Fig. 5

H

702

C

712b

712a

Fig. 7

W

802

816

B

814

812

816

Fig. 8

FIG. 6A

FIG. 6B

FIG. 9

FIG. 10

1102

F

1112

1104

FIG. 11A

1102

F

0.002"

1112

1104

FIG. 11B

1102

F

1112

1104

FIG. 11C

1102

F

1112

1104

FIG. 11D

FIG. 12A

FIG. 12B

FIG. 13

FIG. 14A

FIG. 14B

FIG. 14c

FIG. 15A

FIG. 15B

FIG. 15C

FIG. 16A

FIG. 16B

EP 2 170 432 B1

FIG. 16C

FIG. 16D

FIG. 16E

1605

1654

1610

1652

1602

1656

36

1606

1620

1657

EP 2 170 432 B1

FIG. 16F

EP 2 170 432 B1

FIG. 16G

FIG. 16H

FIG. 17

FIG. 18A

FIG. 18B

FIG. 19A

FIG. 19B

FIG. 20A

FIG. 20B

Fig. 20c

FIG. 20D

EP 2 170 432 B1

FIG. 21A

EP 2 170 432 B1

FIG. 21B

FIG. 21c

FIG. 21D

FIG. 22A

EP 2 170 432 B1

FIG. 22B

FIG. 23A

FIG. 23B

FIG. 24A

FIG. 24B

FiG. 24c

FiG. 24d

FIG. 24E

FIG. 24F

FIG. 24G

FIG. 24H

2600

2602

2810

2615

Fig. 25

2700

| FORMING AN ELONGATED FRAMEWORK OF FLEXIBLE MATERIAL. |
|---|

2710

↓

| FORMING A SHARP HEAD ON A DISTAL END OF THE ELONGATED FRAMEWORK |
|---|

2720

↓

| JACKETING THE ELONGATED FRAMEWORK WITH A FLEXIBLE TUBE TO DEFINE AT LEAST ONE CONDUIT THEREBETWEEN |
|---|

2730

Fig. 26

2800

| ADHERING A FLEXIBLE CONDUIT INSERTION MEDICAL DEVICE TO A TARGET SITE, THE DEVICE INCLUDING A FLEXIBLE MEDICAL DEVICE CONDUIT AND AN INSERTION MECHANISM |
|---|

2810

↓

| INSERTING THE FLEXIBLE MEDICAL DEVICE CONDUIT INTO THE TARGET SITE BY ACTION OF INSERTION MECHANISM |
|---|

2820

Fig. 27

**EP 2 170 432 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02081013 A
- WO 9817337 A
- WO 0205866 A